(19) Europäisches Patentamt
European Patent Office
Office européen des brevets

(11) EP 3 897 527 B1

(12) **EUROPÄISCHE PATENTSCHRIFT**

(45) Veröffentlichungstag und Bekanntmachung des Hinweises auf die Patenterteilung:
**18.09.2024 Patentblatt 2024/38**

(21) Anmeldenummer: **19805182.3**

(22) Anmeldetag: **12.11.2019**

(51) Internationale Patentklassifikation (IPC):
***A61K 8/22** (2006.01)* ***A61K 8/365** (2006.01)*
***A61K 8/49** (2006.01)* ***A61Q 5/10** (2006.01)*

(52) Gemeinsame Patentklassifikation (CPC):
**A61K 8/22; A61K 8/365; A61K 8/4946; A61K 8/4973; A61Q 5/10;** A61K 2800/4324; A61K 2800/88

(86) Internationale Anmeldenummer:
**PCT/EP2019/080970**

(87) Internationale Veröffentlichungsnummer:
**WO 2020/126225 (25.06.2020 Gazette 2020/26)**

# (54) OXIDATIONSFÄRBEMITTEL IN ROTNUANCEN MIT VERBESSERTER ECHTHEIT UND VERBESSERTER HOMOGENITÄT

OXIDATION DYES IN RED SHADES WITH IMPROVED COLOUR FASTNESS AND IMPROVED HOMOGENEITY

DES COLORANTS D'OXYDATION DANS DES TEINTES ROUGES AVEC UNE MEILLEURE SOLIDITÉ ET HOMOGÉNÉITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.12.2018 DE 102018221959**

(43) Veröffentlichungstag der Anmeldung:
**27.10.2021 Patentblatt 2021/43**

(73) Patentinhaber: **Henkel AG & Co. KGaA**
**40589 Düsseldorf (DE)**

(72) Erfinder:
- **HODES, Jing**
  **58093 Hagen (DE)**
- **HILBIG, Sandra**
  **44894 Bochum (DE)**
- **KESSLER-BECKER, Daniela**
  **51371 Leverkusen (DE)**
- **MOCH, Melanie**
  **41542 Dormagen (DE)**

(56) Entgegenhaltungen:
**EP-A2- 1 875 892 DE-A1- 102006 017 901**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Gegenstand der vorliegenden Anmeldung ist ein Mittel zum oxidativen Färben von keratinischen Fasern, insbesondere menschlichen Haaren, das in einem kosmetischen Träger (A) mindestens ein Oxidationsfarbstoffvorprodukt, ausgewählt aus 4,5-Diamino-1-(2-hydroxyethyl)-1H-pyrazol und/oder einem seiner physiologisch verträglichen Salze und weiterhin (B) Glucoheptonsäure und/oder mindestens eines ihrer physiologisch verträglichen Salze und/oder Lactone enthält. Das erfindungsgemäße Mittel enthält weiterhin (C) mindestens ein Oxidationsmittel, das von Luftsauerstoff verschieden ist.

Ein zweiter Gegenstand der vorliegenden Erfindung ist eine Mehrkomponenten-Verpackungseinheit (Kit-of-parts), die mindestens zwei getrennt voneinander konfektionierte Komponenten umfasst, wobei die erste Komponente (K1) die zuvor beschriebenen Inhaltsstoffe (A) und (B) und die zweite Komponente (K2) den zuvor beschriebenen Inhaltsstoff (C) enthält.

Ein dritter Gegenstand der vorliegenden Erfindung ist eine Mehrkomponenten-Verpackungseinheit (Kit-of-parts), die mindestens zwei getrennt voneinander konfektionierte Komponenten umfasst, wobei die erste Komponente (K1) den zuvor beschriebenen Inhaltsstoff (A) und optional mindestens ein Oxidationsmittel enthält, das ausgewählt ist aus Persalzen, insbesondere Peroxodisulfatsalzen und/oder Peroxomonosulfatsalzen, und die zweite Komponente (K2) den zuvor beschriebenen Inhaltsstoff (B) enthält.

Ein vierter Gegenstand der vorliegenden Erfindung ist eine Mehrkomponenten-Verpackungseinheit (Kit-of-parts), die mindestens drei getrennt voneinander konfektionierte Komponenten umfasst, wobei die erste Komponente (K1) den zuvor beschriebenen Inhaltsstoff (A), die zweite Komponente (K2) den zuvor beschriebenen Inhaltsstoff (C) und die dritte Komponente (K3) den zuvor beschriebenen Inhaltsstoff (B) enthält.

Ein weiterer Gegenstand der vorliegenden Erfindung ist ein Verfahren zur oxidativen Haarfärbung, bei dem ein Mittel zum oxidativen Färben von keratinischen Fasern, insbesondere menschlichen Haaren, auf die Fasern, insbesondere menschliche Haare, aufgetragen wird, das in einem kosmetischen Träger (A) mindestens ein Oxidationsfarbstoffvorprodukt, ausgewählt aus 4,5-Diamino-1-(2-hydroxyethyl)-1H-pyrazol und/oder einem seiner physiologisch verträglichen Salze, weiterhin (B) Glucoheptonsäure und/oder mindestens eines ihrer physiologisch verträglichen Salze und/oder Lactone sowie weiterhin (C) mindestens ein Oxidationsmittel enthält, das von Luftsauerstoff verschieden ist, wobei das Färbemittel nach einer Einwirkzeit von 1 bis 60 Minuten mit Wasser ausgespült und das Haar optional mit weiteren Reinigungs- und Pflegeprodukten behandelt und dann getrocknet wird.

Die Veränderung der Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Zur Farbveränderung der Haare kennt der Fachmann verschiedene Möglichkeiten. Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Die Färbung mit direktziehenden Farbstoffen ist mit einer geringen Haarschädigung verbunden, ein Nachteil ist jedoch die geringe Haltbarkeit und die schnelle Auswaschbarkeit der mit direktziehenden Farbstoffen erhaltenen Färbungen.

Wünscht sich der Verbraucher ein lang anhaltendes Farbergebnis oder eine Nuance, die heller als seine Ausgangshaarfarbe ist, werden üblicherweise oxidative Farbveränderungsmittel eingesetzt. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, so genannte Entwicklerkomponenten (Oxidationsbasen) und Kupplerkomponenten, die unter dem Einfluss von Oxidationsmitteln untereinander die eigentlichen Farbstoffe ausbilden. Oxidationsfärbemittel zeichnen sich durch lang anhaltende Färbeergebnisse aus.

Zu oxidativen Färbemitteln existiert bereits umfangreicher Stand der Technik. Insbesondere zur Optimierung der Farbintensität und der Echtheitseigenschaften von Modenuancen wurden schon viele Versuche unternommen.

EP1875892 (D1) betrifft Mittel zum Färben und/oder Aufhellen keratinischer Fasern, d.h. Mittel zur Anwendung auf Keratinfasern, insbesondere menschlichen Haaren, und deren Verwendung.

DE10 2006 017 901 (D2) betrifft ebenfalls Mittel zum Färben und/oder Aufhellen keratinischer Fasern.

Doch trotz der großen Anzahl der bereits durchgeführten Optimierungsversuche besteht im Hinblick auf die Echtheitseigenschaften der Färbung von oxidativ gefärbten Keratinfasern - insbesondere wenn diese in einer Modenuance im Rotbereich gefärbt werden sollen - immer noch Verbesserungsbedarf. Vor allem die Waschechtheit von Rotnuancen auf der Basis von 4,5-Diaminopyrazolen als Entwicklerkomponente (Oxidationsbase) kann noch nicht als optimal eingestuft werden.

Die Aufgabe der vorliegenden Erfindung war die Bereitstellung von oxidativen Färbemitteln zur Erzielung von Rotnuancen auf der Basis von 4,5-Diaminopyrazolen als Entwicklerkomponente mit verbesserten Echtheitseigenschaften, insbesondere mit verbesserter Waschechtheit.

Unter der Waschechtheit einer Farbnuance wird die farbliche Veränderung der mit dieser Nuance gefärbten Haarsträhne unter dem Einfluss von mehreren Haarwäschen verstanden. Bei dieser farblichen Veränderung kann es sich sowohl um eine Verschiebung der Farbe in Richtung eines anderen Farbtons als auch um das Verblassen der

Färbung handeln. Beide Farbänderungen sind vom Anwender gleichermaßen unerwünscht. Farbnuancen mit guter Waschechtheit verändern sich farblich auch nach wiederholten Haarwäschen nicht oder kaum. Die Haarwäsche kann hierbei unter Zuhilfenahme eines Shampoos, eines konditionierenden Shampoos oder auch eines Konditioners erfolgen.

Ein weiteres Problem, dass es bei der Formulierung von oxidativen Haarfärbemitteln zu bewältigen gilt, betrifft die Homogenität des Färbeergebnisses auf dem Haar. Von der Haarwurzel bis zur Haarspitze ist die Haarfaser verschieden stark geschädigt. Haare im Bereich des Ansatzes sind gerade frisch nachgewachsen und wurden noch keinen oder nur geringen Witterungseinflüssen, chemischen (Färben, Blondieren, Dauerwellen, Waschen, Schwimmbadwasser) oder physikalischen (Kämmen, Föhnen) Einflüssen ausgesetzt. Der Bereich der Haarlängen ist umso geschädigter, je weiter entfernt er vom Haaransatz und somit je älter er ist. Die Haare im Bereich der Spitzen sind die ältesten Teile des Haares und weisen daher die stärkste Schädigung auf.

In geschädigtem Haar ist die Cuticula, die Schuppenschicht des Haares, in mehr oder weniger starkem Ausmaß zerstört. Dies führt dazu, dass auf geschädigtem Haar generell ein stärkerer Farbaufzug stattfindet. Wenn Ansatz und Spitzen mit demselben Färbemittel gefärbt werden, besteht bei stärker geschädigtem Haar daher immer die Gefahr eines ungleichmäßigen Farbergebnisses.

Im Sinne der vorliegenden Anmeldung gelten die Haarspitzen als Teil der Haarlängen. Ist von Haarlängen die Rede, so sind immer auch die Haarspitzen eingeschlossen.

Unter dem Haaransatz werden erfindungsgemäß die direkt an der Kopfhaut befindliche Haarpartie (die ersten 0 bis 5 cm des Haares) verstanden.

Unter dem Bereich der Haarlängen wird dementsprechend der Bereich der Haarfaser verstanden, der mehr als 5 cm von der Kopfhaut entfernt ist. Unter dem Bereich der der Haarspitzen werden erfindungsgemäß die letzten 3 cm der Haarfaser verstanden.

Es war eine weitere Aufgabe der vorliegenden Erfindung, dem Friseur oder dem Heimanwender ein oxidatives Haarfärbemitteln zur Verfügung zu stellen, mit dem sich entlang der Haarfaser eine möglichst homogene, gleichmäßige Färbung erzielen lässt.

Zur Verbesserung der Homogenität einer Färbung bzw. der Verringerung der Selektivität eines Färbemittels bzw. der Verbesserung des Ausgleichsvermögens eines Färbemittels kennt der Fachmann weitere Methoden. EP 2471504A1 beschäftigt sich mit der Aufgabe, oxidative Haarfärbemittel mit hohem Ausgleichsvermögen bereitzustellen, die sowohl auf den geschädigten Partien der Haarfaser als auch auf dem ungeschädigten oder wenig geschädigten Haaransatz eine einheitliche Haarfärbung erzielen. EP 2471504A1 löst diese Aufgabe mit einer Wirkstoffkombination aus einem Aminosäuretensid, einem Kationtensid und einem Öl.

Keratinische Fasern können in Rottönen gefärbt werden, wenn in den Färbemitteln (A) mindestens ein Oxidationsfarbstoffvorprodukt der Struktur (I) und/oder eines seiner physiologisch verträglichen Salze

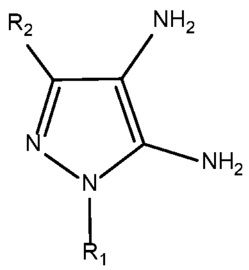

(I)

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, als Entwicklerkomponente bzw. Oxidationsbase enthalten ist.

Erfindungsgemäß ist das Oxidationsfarbstoffvorprodukt der Struktur (I), in der $R_1$ für eine 2-Hydroxyethyl-Gruppe und $R_2$ für Wasserstoff stehen, also 4,5-Diamino-1-(2-hydroxyethyl)-1H-pyrazol und/oder eines seiner physiologisch verträglichen Salze, bevorzugt 4,5-Diamino-1-(2-hydroxyethyl)-1H-pyrazolsulfat.

Die Entwicklerkomponente 4,5-Diamino-1-(2-hydroxyethyl)-1H-pyrazol wird seit langem in vielen Oxidationsfärbemitteln eingesetzt. Entsprechende Mittel sind beispielsweise aus EP 1321131A2 bekannt. Dieses Dokument lehrt die Verwendung von einem N-haltigen Silikon, Polystyrolsulfonat und/oder Pyrrolidon zur Verbesserung der Waschechtheit von oxidativen Haarfärbungen, die ein Oxidationsfarbstoffvorprodukt wie 4,5-Diamino-1-(2-hydroxyethyl)-1H-pyrazol enthalten.

Andererseits weiß man, dass Polymere, wie N-haltige Silikone oder Polystyrolsulfonat, einen Farbverschiebenden Einfluss auf die Ausfärbung haben können in dem Sinne, dass gegenüber der Färbemittel-Basis ohne den Echtheits verbessernden Zusatz ein Farbshift des Färbeergebnisses auftritt. Solche Farbverschiebungen sind aus technischen und ökonomischen Gründen unerwünscht.

Pyrrolidon bildet oberhalb seines Flammpunkts von 138°C entzündliche Dampf-Luft-Gemische und ist daher ein für die großtechnische Herstellung eines Kosmetikproduktes weniger bevorzugter Inhaltsstoff.

Es bestand somit die technische Aufgabe, alternative Oxidationsfärbemittel mit 4,5-Diaminopyrazolen als Entwicklerkomponente für Rotnuancen mit hoher Waschechtheit bereitzustellen, deren Echtheits verbessernder Zusatzstoff keine oder nur sehr geringe Farbverschiebungen bewirkt, sich großtechnisch problemlos verarbeiten lässt und möglichst ökologisch unbedenklichen Rohstoffen basiert.

Eine weitere technische Aufgabe bestand darin, alternative Oxidationsfärbemittel mit 4,5-Diaminopyrazolen als Entwicklerkomponente für Rotnuancen mit großer Homogenität bzw. geringer Selektivität der Färbung entlang der gesamten Haarfaser bereitzustellen, deren Homogenität verbessernder Zusatzstoff keine oder nur sehr geringe Farbverschiebungen bewirkt, sich großtechnisch problemlos verarbeiten lässt und möglichst ökologisch unbedenklichen Rohstoffen basiert.

Überraschenderweise wurde gefunden, dass mit der Kombination aus (A) mindestens einem Oxidationsfarbstoffvorprodukt mit 4,5-Diaminopyrazol als Basisstrukturelement gemäß der Strukturformel (I), ausgewählt aus 4,5-Diamino-1-(2-hydroxyethyl)-1H-pyrazol und/oder einem seiner physiologisch verträglichen Salze, und weiterhin (B) Glucoheptonsäure und/oder mindestens eines ihrer physiologisch verträglichen Salze und/oder Lactone einem kosmetischen Träger Oxidationsfärbemittel erhalten werden, die attraktive und modische Rotnuancen mit hoher Waschechtheit bereitstellen, dabei umkompliziert herzustellen sind und auf ökologisch unbedenklichen Rohstoffen basieren.

Oxidationsfärbemittel mit einem Gehalt an Glucoheptonsäure oder Natriumglucoheptonat (INCI: sodium gluceptate) sind im Stand der Technik bekannt aus WO 2006/106366A1 und WO 2003/015734A1, weiterhin auch aus JP2004210700A. Entsprechende Marktprodukte sind offenbart bei Mintel GNPD, Eintragsnummern 928580, 667942, 298707 und 28770. Keines dieser Dokumente offenbart einen Gehalt an 4,5-Diaminopyrazolen oder die Verwendung von Glucoheptonsäure oder Natriumglucoheptonat zur Verbesserung der Waschechtheit.

Ein erster Gegenstand der vorliegenden Erfindung ist daher ein Mittel zum oxidativen Färben von keratinischen Fasern, insbesondere menschlichen Haaren, enthaltend in einem kosmetischen Träger

(A) mindestens ein Oxidationsfarbstoffvorprodukt der Struktur (I) und/oder eines seiner physiologisch verträglichen Salze

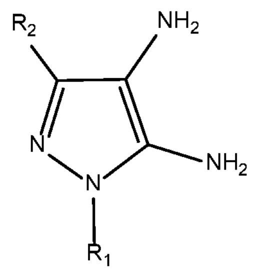

(I),

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, weiterhin

(B) Glucoheptonsäure und/oder mindestens eines ihrer physiologisch verträglichen Salze und/oder Lactone, und

(C) mindestens ein Oxidationsmittel, das von Luftsauerstoff verschieden ist, dadurch gekennzeichnet, dass es mindestens ein Oxidationsfarbstoffvorprodukt der Struktur (I) enthält, in der $R_1$ für eine 2-Hydroxyethyl-Gruppe und $R_2$ für Wasserstoff stehen.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Unter dem erfindungsgemäß verwendeten Begriff "Mittel zur oxidativen Färben" werden oxidative Färbemittel verstanden, die Oxidationsfarbstoffvprodukte vom Entwickertyp und vom Kupplertyp enthalten. Die Ausbildung der Färbung erfolt durch die Anwesenheit eines Oxidationsmittels (C), das von Luftsauerstoff verschieden ist und bei dem es sich bevorzugt um Wasserstoffperoxid handelt. Abhängig von der Menge des eingesetzten Oxidationsmittels wird die Keratinfaser während der Färbung gleichzeitig mehr oder weniger stark aufgehellt, da das Oxidationsmittel nicht nur den Farbstoffbildungsprozess von Entwicklern und Kupplern initiiert, sondern auch die haareigenen Pigmente (Melanine) oxidativ zerstört.

Die erfindungsgemäßen Mittel enthalten das mindestens eine Oxidationsfarbstoffvorprodukt (A) der Struktur (I), ausgewählt aus 4,5-Diamino-1-(2-hydroxyethyl)-1 H-pyrazol und/oder einem seiner physiologisch verträglichen Salze, sowie die Komponente (B) und das Oxidationsmittel (C) in einem kosmetischen Träger, bevorzugt in einem geeigneten wässrigen, alkoholischen oder wässrigalkoholischen Träger. Zum Zweck der oxidativen Färbung können solche Träger beispielsweise Cremes, Emulsionen, Gele oder auch schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei Mitteln zum oxidativen Färben von keratinischen Fasern um Cremes oder Emulsionen.

Kennzeichnend für die erfindungsgemäßen Mittel ist ein Gehalt an mindestens einem Oxidationsfarbstoffvorprodukt (A) der Struktur (I) und/oder einem seiner physiologisch verträglichen Salze

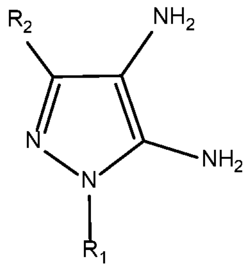

(I),

worin $R_1$ und $R_2$ unabhängig voneinander stehen für Wasserstoff oder eine lineare oder verzweigte $C_1$-$C_{10}$-Alkylgruppe, die mit ein bis zehn Hydroxyl-Gruppen substituiert sein kann, wobei $R_1$ und $R_2$ nicht gleichzeitig für Wasserstoff stehen, wobei mindestens ein Oxidationsfarbstoffvorprodukt der Struktur (I) enthalten ist, in der $R_1$ für eine 2-Hydroxyethyl-Gruppe und $R_2$ für Wasserstoff stehen.

Unter einem Entwickler wird im Sinne der vorliegenden Erfindung ein Oxidationsfarbstoffvorprodukt vom Entwicklertyp verstanden. Unter einem Kuppler wird im Sinne der vorliegenden Erfindung ein Oxidationsfarbstoffvorprodukt vom Kupplertyp verstanden.

Als Oxidationsfarbstoffvorprodukt (A) enthalten erfindungsgemäße Mittel das 4,5-Diamino-1-(2-hydroxyethyl)-1H-pyrazol und/oder eines seiner physiologisch verträglichen Salze.

Bei 4,5-Diamino-1-(2-hydroxyethyl)-1H-pyrazol handelt es sich um die Verbindung der Formel (I-A), also ein Oxidationsfarbstoffvorprodukt der Struktur (I), in der $R_1$ für eine 2-Hydroxyethyl-Gruppe und $R_2$ für Wasserstoff stehen, also 4,5-Diamino-1-(2-hydroxyethyl)-1H-pyrazol und/oder eines seiner physiologisch verträglichen Salze:

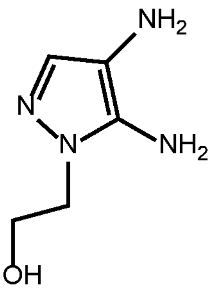

(I-A) molare Masse = 142,16 g/mol.

Bevorzugte physiologisch verträgliche Salze von 4,5-Diamino-1-(2-hydroxyethyl)-1H-pyrazol sind insbesondere die Hydrochloride (Monohydrochlorid × HCl, oder Dihydrochlorid × 2 HCl), das Sulfat (× $H_2SO_4$) und die Hydrobromide (Monohydrobromid × HBr, oder Dihydrobromid × 2 HBr) der Verbindung. Ganz besonders bevorzugt ist 4,5-Diamino-1-(2-hydroxyethyl)-1H-pyrazolsulfat (Formel (III)).

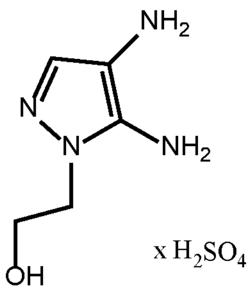

x $H_2SO_4$

(III) molare Masse = 240,23 g/mol

Erfindungsgemäß bevorzugte Mittel zum oxidativen Färben sind dadurch gekennzeichnet, dass das mindestens eine Oxidationsfarbstoffvorprodukt (A) der Struktur (I) in einer Gesamtmenge von 0,01 bis 2,5 Gew.-%, bevorzugt 0,1 bis 1,8 Gew.-%, besonders bevorzugt 0,2 bis 1,0 Gew.-%, außerordentlich bevorzugt 0,4 bis 0,9 Gew.-%, enthalten ist, wobei sich die Mengenangaben auf das Gewicht der freien 4,5-Diaminopyrazol-Base in Relation zum Gewicht des erfindungsgemäßen Mittels beziehen.

Erfindungsgemäß besonders bevorzugte Mittel zum oxidativen Färben sind dadurch gekennzeichnet, dass 4,5-Diamino-1-(2-hydroxyethyl)-1H-pyrazol (Struktur I-A) in einer Gesamtmenge von 0,01 bis 2,5 Gew.-%, bevorzugt 0,1 bis 1,8 Gew.-%, besonders bevorzugt 0,2 bis 1,0 Gew.-%, außerordentlich bevorzugt 0,4 bis 0,9 Gew.-%, enthalten ist, wobei sich die Mengenangaben auf das Gewicht der freien 4,5-Diaminopyrazol-Base in Relation zum Gewicht des erfindungsgemäßen Mittels beziehen.

4,5-Diamino-1-(2-hydroxyethyl)-1H-pyrazol (I-A) wird ganz besonders bevorzugt in Form des Sulfat-Salzes in einer Menge von 0,025 bis 4,5 Gew.-%, bevorzugt 0,1 bis 3,5 Gew.-%, weiter bevorzugt 0,2 bis 2,0 Gew.-% und ganz besonders bevorzugt 0,3 bis 1,0 Gew.-% eingesetzt. Hierbei ist die Mengenangabe auf das Gewicht von 4,5-Diamino-1-(2-hydroxyethyl)-1H-pyrazolsulfat (Formel (III)) bezogen, das zum Gewicht des erfindungsgemäßen Mittels in Relation gesetzt wird.

In einer besonders bevorzugten Ausführungsform ist ein erfindungsgemäßes Mittel daher dadurch gekennzeichnet, dass es - bezogen auf sein Gesamtgewicht - (A) von 0,025 bis 4,5 Gew.-%, bevorzugt 0,1 bis 3,5 Gew.-%, weiter bevorzugt 0,2 bis 2,0 Gew.-% und ganz besonders bevorzugt 0,3 bis 1,0 Gew.-% 4,5-Diamino-1-(2-hydroxyethyl)-1H-pyrazolsulfat enthält.

Als zweiten wesentlichen Bestandteil (B) enthalten die erfindungsgemäßen Mittel Glucoheptonsäure und/oder mindestens eines ihrer physiologisch verträglichen Salze und/oder Lactone.

Glucoheptonsäure (226,18 g/mol) wird auch als D-*glycero*-D-*gulo*-Heptonsäure bezeichnet.

Zu den physiologisch verträglichen Salzen der Glucoheptonsäure, die im Rahmen der vorliegenden Erfindung zählen insbesondere die Salze von Alkalimetallen, Erdalkalimetallen und Erdmetallen, insbesondere von Lithium, Natrium, Kalium, Magnesium und Calcium, besonders bevorzugt Natrium und Kalium, außerordentlich bevorzugt Natrium. Das erfindungsgemäß außerordentlich bevorzugte Natriumglucoheptonat (INCI: sodium gluceptate; 248 g/mol) ist im Handel erhältlich.

Zu den erfindungsgemäß bevorzugten Lactonen der Glucoheptonsäure zählen das 1,4-Lacton (Schmelzpunkt 151°C) und das 1,5-Lacton, wobei das 1,4-Lacton außerordentlich bevorzugt ist.

Erfindungsgemäß bevorzugte Mittel zum oxidativen Färben sind dadurch gekennzeichnet, dass Glucoheptonsäure und/oder mindestens eines ihrer physiologisch verträglichen Salze und/oder Lactone in einer Gesamtmenge von 0,01 bis 2,5 Gew.-%, bevorzugt 0,05 bis 1,5 Gew.-%, besonders bevorzugt 0,1 bis 1,0 Gew.-%, außerordentlich bevorzugt 0,1 bis 0,5 Gew.-% enthalten ist/sind, wobei sich die Mengenangaben auf das Gewicht an freier Glucoheptonsäure in Relation zum Gewicht des erfindungsgemäßen Mittels beziehen.

Als dritten wesentlichen Bestandteil enthält das erfindungsgemäße Mittel zum oxidativen Färben von keratinischen Fasern (C) mindestens ein Oxidationsmittel, das von Luftsauerstoff verschieden ist.

Erfindungsgemäß bevorzugte Oxidationsmittel (C) sind ausgewählt aus Wasserstoffperoxid.

Sobald das mindestens eine Oxidationsfarbstoffvorprodukt (A) und gegebenenfalls weitere optional enthaltene Oxidationsfarbstoffvorprodukte mit dem Oxidationsmittel (C) in Gegenwart von Wasser in Kontakt kommen, startet ein Kupplungsprozess und die Farbstoffbildung beginnt.

Bei dem erfindungsgemäßen Mittel, das die Inhaltsstoffe (A), (B) und (C) enthält, handelt es sich somit um das anwendungsbereite oxidative Färbemittel, sofern das Oxidationsmittel (C) Wasserstoffperoxid umfasst.

Die Menge an Oxidationsmittel wird der Fachmann in Abhängigkeit von der gewünschten Aufhellleistung wählen. Wenn die Ausbildung einer sehr dunklen Rotnuance gewünscht wird, wird der Fachmann die Einsatzmenge an Wasserstoffperoxid entsprechend reduzieren. Soll jedoch eine leuchtende Rotnuance auf dunklem Haar erzielt werden, muss das Haar gleichzeitig auch in einem signifikanten Ausmaß aufgehellt werden. In diesem Fall wird die Einsatzmenge an Wasserstoffperoxid entsprechend hoch gewählt; gegebenenfalls kann für diesen Fall als weiteres Oxidationsmittel (C) ein Persalz, insbesondere ein oder mehrere Peroxodisulfatsalze und/oder Peroxomonosulfatsalze, wie Kaliumpersulfat, Natriumpersulfat oder Ammoniumpersulfat, enthalten sein.

Erfindungsgemäß bevorzugte Mittel sind dadurch gekennzeichnet, dass sie, jeweils bezogen auf ihr Gewicht, 0,5 bis 12 Gew.-%, bevorzugt 0,9 bis 7 Gew.-%, besonders bevorzugt 1,5 bis 5 Gew.-%, außerordentlich bevorzugt 3 bis 4,5 Gew.-% Wasserstoffperoxid (berechnet als 100 %-iges $H_2O_2$) als Oxidationsmittel (C) enthalten.

Bei dem vorangehend beschriebenen erfindungsgemäßen Mittel, das Wasserstoffperoxid als Oxidationsmittel (C) enthält, handelt es sich um das anwendungsbereite Mittel, das bereits sowohl mindestens ein Oxidationsfarbstoffvorprodukt (A) als auch ein Oxidationsmittel (C) enthält, das den Kupplungsprozess des oder der Oxidationsfarbstoffvorprodukte/s miteinander und die damit einhergehende Farbstoffbildung bewirkt, nämlich Wasserstoffperoxid. Zur Herstellung dieses anwendungsbereiten Färbemittels und zur Initiierung der Farbstoffbildungsreaktion wird in der Regel eine erste Komponente K1, die das mindestens ein Oxidationsfarbstoffvorprodukt (A) und optional weitere Oxidationsfarbstoffvorprodukte (OFV) enthält, aber frei ist von gelöstem Wasserstoffperoxid, mit einer zweiten Komponente K2, die Wasserstoffperoxid als Oxidationsmittel (C) enthält, vermischt. Das so erhaltene anwendungsbereite Färbemittel ist zur sofortigen Applikation auf das zu färbende Haar bestimmt. Der mindestens eine erfindungsgemäße Wirkstoff (B), also Glucoheptonsäure und/oder mindestens eines ihrer physiologisch verträglichen Salze und/oder Lactone, kann in Komponente K1 oder in Komponente K2 enthalten sein. Für die großtechnische Fertigung der Komponenten eines Oxidationsfärbemittels kann es bevorzugt sein, die Wasserstoffperoxid-haltige Komponente K2 in möglichst wenig komplexer, standardisierter Zusammensetzung zu formulieren, das heißt, den erfindungsgemäßen Wirkstoff (B), also Glucoheptonsäure und/oder mindestens eines ihrer physiologisch verträglichen Salze und/oder Lactone, in der OFV-haltigen Komponente K1 bereitzustellen.

Zur Vermeidung von Inkompatibilitäten und zur Verhinderung einer vorzeitigen, unerwünschten Farbstoffbildung werden die Komponenten K1 (Oxidationsfarbstoffvorprodukt-haltig) und K2 (Oxidationsmittelzubereitung mit gelöstem Wasserstoffperoxid) stets getrennt voneinander konfektioniert und erst kurz vor der Anwendung miteinenander in Kontakt gebracht. Für den Verbraucher werden die Komponenten (K1) und (K2) bevorzugt in Form einer Mehrkomponenten-Verpackungseinheit (Kit-of-parts) bereitgestellt.

Ein zweiter Gegenstand der vorliegenden Erfindung ist daher eine Mehrkomponenten-Verpackungseinheit (Kit-of-parts) zur oxidativen Färbung keratinischer Fasern, umfassend mindestens zwei getrennt voneinander konfektionierte Komponenten (K1) und (K2), wobei

- die erste Komponente (K1) in einem kosmetischen Träger

  (A) mindestens ein Oxidationsfarbstoffvorprodukt der Struktur (I) und/oder eines seiner physiologisch verträglichen Salze enthält

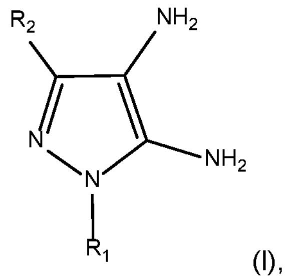

(I),

  worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, wobei die Komponente (A) gemäß Anspruch 1 ausgewählt ist, weiterhin
  (C) optional mindestens ein Oxidationsmittel, das ausgewählt ist aus Persalzen, insbesondere Peroxodisulfatsalzen und/oder Peroxomonosulfatsalzen,

- die zweite Komponente (K2)
  (C) Wasserstoffperoxid enthält, das in Wasser gelöst ist,

dadurch gekennzeichnet, dass mindestens eine der Komponenten (K1) oder (K2) (B) Glucoheptonsäure und/oder mindestens eines ihrer physiologisch verträglichen Salze und/oder Lactone enthält.

Für die großtechnische Fertigung der Komponenten eines Oxidationsfärbemittels kann es bevorzugt sein, die Wasserstoffperoxid-haltige Komponente K2 in möglichst wenig komplexer, standardisierter Zusammensetzung zu formulieren, das heißt, den erfindungsgemäßen Wirkstoff (B), also Glucoheptonsäure und/oder mindestens eines ihrer physiologisch verträglichen Salze und/oder Lactone, in der OFV-haltigen Komponente K1 bereitzustellen.

Erfindungsgemäß bevorzugt ist daher eine Mehrkomponenten-Verpackungseinheit (Kit-of-parts) zur oxidativen Färbung keratinischer Fasern, umfassend mindestens zwei getrennt voneinander konfektionierte Komponenten (K1) und (K2), wobei

- die erste Komponente (K1) in einem kosmetischen Träger

  (A) mindestens ein Oxidationsfarbstoffvorprodukt der Struktur (I) und/oder eines seiner physiologisch verträglichen Salze enthält

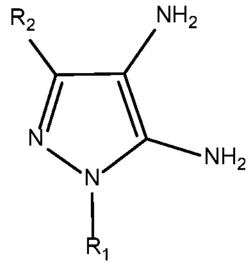

(I),

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, wobei die Komponente (A) gemäß Anspruch 1 ausgewählt ist, weiterhin

(B) Glucoheptonsäure und/oder mindestens eines ihrer physiologisch verträglichen Salze und/oder Lactone, weiterhin

(C) optional mindestens ein Oxidationsmittel enthält, das ausgewählt ist aus Persalzen, insbesondere Peroxodisulfatsalzen und/oder Peroxomonosulfatsalzen,

- die zweite Komponente (K2)
  (C) Wasserstoffperoxid enthält, das in Wasser gelöst ist.

Das, was hinsichtlich bevorzugter Oxidationsfarbstoffvorprodukte (A) der Struktur (I), bevorzugter qualitativer Ausführungsformen des Inhaltsstoffs (B) und bevorzugter qualitativer Ausführungsformen des Inhaltsstoffs (C) zum ersten Gegenstand der vorliegenden Erfindung gesagt wurde, gilt mutatis mutandis auch für den zweiten Gegenstand der vorliegenden Erfindung. Die erfindungsgemäß bevorzugten Mengen an Inhaltsstoffen (A), (B) und (C), die vorstehend für den ersten Gegenstand der vorliegenden Erfindung offenbart sind, gelten mutatis mutandis auch für die Mischungen aus den Komponenten (K1) und (K2) des zweiten Gegenstands der vorliegenden Erfindung. Die Konzentrationen der Inhaltsstoffe (A), (B) und (C) in den Komponenten (K1) und (K2) und die Mischungsverhältnisse der Komponenten (K1) und (K2) zueinander sind vom Fachmann entsprechend so auszuwählen, dass die Mischungen aus den Komponenten (K1) und (K2) die bevorzugten Mengen an Inhaltsstoffen (A), (B) und (C), die vorstehend für den ersten Gegenstand der vorliegenden Erfindung offenbart sind, aufweisen.

Ein weiterer Gegenstand der vorliegenden Erfindung ist eine Mehrkomponenten-Verpackungseinheit (Kit-of-parts) zur oxidativen Färbung keratinischer Fasern, umfassend mindestens drei getrennt voneinander konfektionierte Komponenten (K1), (K2) und (K3), wobei

- die erste Komponente (K1) in einem kosmetischen Träger

(A) mindestens ein Oxidationsfarbstoffvorprodukt der Struktur (I) und/oder eines seiner physiologisch verträglichen Salze enthält

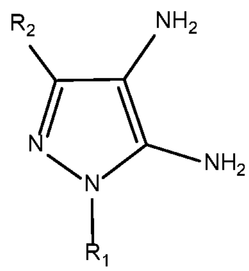

(I),

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, wobei die Komponente (A) gemäß Anspruch 1 ausgewählt ist, weiterhin

(C) optional mindestens ein Oxidationsmittel enthält, das ausgewählt ist aus Persalzen, insbesondere Peroxodisulfatsalzen und/oder Peroxomonosulfatsalzen,

(C) - die zweite Komponente (K2C) Wasserstoffperoxid enthält, das in Wasser gelöst ist,

- die dritte Komponente (K3)

(B) Glucoheptonsäure und/oder mindestens eines ihrer physiologisch verträglichen Salze und/oder Lactone enthält.

Der oben dargestellte vierte Gegenstand der Erfindung sieht vor, dass die Komponente (B), also Glucoheptonsäure und/oder mindestens eines ihrer physiologisch verträglichen Salze und/oder Lactone, getrennt von der OFV-haltigen Komponente (K1) und getrennt von der Wasserstoffperoxid-haltigen Komponente (K2) in einer dritten Komponente (K3) enthalten ist. Das anwendungsbereite Mittel gemäß dieser Ausführungsform wird durch Vermischen der Komponenten (K1), (K2) und (K3) miteinander erhalten. Prinzipiell kann die dritte Komponente (K3) den Wirkstoff (B), also mindestens ein physiologisch verträgliches Salz der Glucoheptonsäure und/oder ein Lacton der Glucoheptonsäure, insbesondere das 1,4-Lacton der Glucoheptonsäure, in reiner, unverdünnter Form enthalten. Glucoheptonsäure selbst ist nur in wässriger Lösung verfügbar, denn beim Eindampfen der wässrigen Lösung kristallisiert das 1,4-Lacton der Glucoheptonsäure aus, das einen Schmelzpunkt von 151°C aufweist. Da die Erfindung im Wesentlichen ein Verbraucherprodukt betrifft, sollte sie so ausgestaltet sein, dass aus den Komponenten (K1), (K2) und (K3) möglichst schnell und mit geringem apparativen Aufwand eine homogene Mischung hergestellt werden kann, die zur unverzüglichen Applikation auf die zu färbenden Fasern geeignet ist. Daher besteht die Komponente (K3) bevorzugt aus einer wässrigen Lösung des erfindungsgemäß verwendeten Wirkstoffs (B), also Glucoheptonsäure und/oder mindestens eines ihrer physiologisch verträglichen Salze und/oder Lactone, wobei diese Lösung optional noch weitere Inhaltsstoffe enthalten kann. Das, was hinsichtlich bevorzugter Oxidationsfarbstoffvorprodukte (A) der Struktur (I), bevorzugter qualitativer Ausführungsformen des Inhaltsstoffs (B) und bevorzugter qualitativer Ausführungsformen des Inhaltsstoffs (C) zum ersten Gegenstand der vorliegenden Erfindung gesagt wurde, gilt mutatis mutandis auch für den vierten Gegenstand der vorliegenden Erfindung. Die erfindungsgemäß bevorzugten Mengen an Inhaltsstoffen (A), (B) und (C), die vorstehend für den ersten Gegenstand der vorliegenden Erfindung offenbart sind, gelten mutatis mutandis auch für die Mischungen aus den Komponenten (K1), (K2) und (K3) des vierten Gegenstands der vorliegenden Erfindung. Die Konzentrationen der Inhaltsstoffe (A), (B) und (C) in den Komponenten (K1), (K2) oder (K3) und die Mischungsverhältnisse der Komponenten (K1), (K2) und (K3) zueinander sind vom Fachmann entsprechend so auszuwählen, dass die Mischungen aus den Komponenten (K1), (K2) und (K3) die bevorzugten Mengen an Inhaltsstoffen (A), (B) und (C), die vorstehend für den ersten Gegenstand der vorliegenden Erfindung offenbart sind, aufweisen.

Bei der ersten Komponente des zweiten, dritten und vierten Gegenstands der Erfindung handelt es sich um die - bevorzugt alkalisch eingestellte - Färbezubereitung (K1), die das mindestens eine Oxidationsfarbstoffvorprodukt (A) der Struktur (I), ausgewählt aus 4,5-Diamino-1-(2-hydroxyethyl)-1H-pyrazol und/oder einem seiner physiologisch verträglichen Salze, sowie gegebenenfalls zusätzliche Oxidationsfarbstoffvorprodukte und/oder weitere direktziehende Farbstoffe enthält.

Vor der Anwendung wird diese Färbezubereitung mit einer Oxidationsmittelzubereitung (K2) vermischt. Die Oxidationsmittelzubereitung (K2) ist aus Stabilitätsgründen bevorzugt auf einen sauren pH-Wert eingestellt und enthält das Oxidationsmittel. Bei der Oxidationsmittelzubereitung (K2) des ersten, zweiten, dritten und vierten Gegenstands der Erfindung handelt es sich um Wasserstoffperoxid, das in Form einer wässrigen Lösung eingesetzt wird.

Die Komponenten (K1) und (K2) können in unterschiedlichen Gewichtsverhältnissen (K1)/(K2) von beispielsweise 0,3 bis 3,0, bevorzugt von 0,5 bis 2,5, besonders bevorzugt von 0,45 bis 1,5 und außerordentlich bevorzugt im Gewichtsverhältnis 1:1 miteinander vermischt werden.

Ein besonders bevorzugtes Verfahren zur oxidativen Haarfärbung ist daher dadurch gekennzeichnet, dass die erste Komponente (K1) und die zweite Komponente (K2) in einem Gewichtsverhältnis (K1)/(K2) von 0,3 bis 3,0, bevorzugt von 0,45 bis 2,5, besonders bevorzugt von 0,45 bis 1,5 und außerordentlich bevorzugt im Gewichtsverhältnis 1:1 miteinander vermischt werden.

Der kometische Träger für die Komponente (K1), die das mindestens eine Oxidationsfarbstoffvorprodukt (A) mit 4,5-Diaminopyrazol als Basisstrukturelement gemäß der Strukturformel (I), ausgewählt aus 4,5-Diamino-1-(2-hydroxyethyl)-1H-pyrazol und/oder einem seiner physiologisch verträglichen Salze, bis zur Herstellung des anwendungsbereiten Färbemittels enthält, kann als Wasser-basierte Emulsion, als Spray, als Creme, Gel, Lotion, Paste oder Shampoo formuliert sein.

Weitere bevorzugte erfindungsgemäße Oxidationsfärbemittel sind dadurch gekennzeichnet, dass sie mindestens ein lineares gesättigtes Alkanol mit 12 - 30 Kohlenstoffatomen enthalten. Im Sinne der vorliegenden Erfindung werden Alkanole mit mindestens 8 Kohlenstoffatomen als Fettsubstanzen angesehen, nicht als Tenside.

Bevorzugte lineare gesättigte Alkanole mit 12 - 30 Kohlenstoffatomen, insbesondere mit 16 - 22 Kohlenstoffatomen, sind ausgewählt aus Cetylalkohol, Stearylalkohol, Arachidylalkohol, Behenylalkohol und Lanolinalkohol sowie Mischungen dieser Alkanole. Erfindungsgemäß besonders bevorzugte Alkanolmischungen sind solche, wie sie bei der technischen Hydrierung von pflanzlichen und tierischen Fettsäuren erhältlich sind. Bevorzugt beträgt die Gesamtmenge an mindestens einem linearen gesättigten Alkanol mit 12 - 30 Kohlenstoffatomen im erfindungsgemäßen Oxidationsfärbemittel 0,1 - 20 Gew.-%, bevorzugt 0,5 - 16,5 Gew.-% und besonders bevorzugt 3 - 10 Gew.-%, jeweils bezogen auf das Gewicht des anwendungsbereiten Oxidationsfärbemittels. Weiterhin bevorzugt beträgt die Gesamtmenge an mindestens einem linearen gesättigten Alkanol mit 12 - 30 Kohlenstoffatomen in der OFV-haltigen Komponente (K1) des erfindungsgemäßen Oxidationsfärbemittels 0,1 - 20 Gew.-%, bevorzugt 0,5 - 16,5 Gew.-% und besonders bevorzugt

3 - 10 Gew.-%, jeweils bezogen auf das Gewicht der Komponente (K1) des erfindungsgemäßen Oxidationsfärbemittels.

Weitere bevorzugte erfindungsgemäße Oxidationsfärbemittel enthalten mindestens ein Tensid oder einen Emulgator.

Tenside und Emulgatoren im Sinne der vorliegenden Anmeldung sind amphiphile (bifunktionelle) Verbindungen, die aus mindestens einem hydrophoben und mindestens einem hydrophilen Molekülteil bestehen. Der hydrophobe Rest ist bevorzugt eine Kohlenwasserstoffkette mit 8-28 Kohlenstoff-Atomen, die gesättigt oder ungesättigt, linear oder verzweigt sein kann. Besonders bevorzugt ist diese $C_8$-$C_{28}$-Alkylkette linear. Basiseigenschaften der Tenside und Emulgatoren sind die orientierte Absorption an Grenzflächen sowie die Aggregation zu Mizellen und die Ausbildung von lyotropen Phasen.

Bei der Auswahl erfindungsgemäß geeigneter Tenside kann es bevorzugt sein, ein Gemisch von Tensiden einzusetzen, um die Stabilität der erfindungsgemäßen Oxidationsfärbemittel optimal einzustellen.

Bevorzugt beträgt die Gesamtmenge an mindestens einem Tensid in den erfindungsgemäßen Oxidationsfärbemitteln 0,1 - 20 Gew.-%, bevorzugt 0,5 - 10 Gew.-% und besonders bevorzugt 1,5 - 5 Gew.-%, jeweils bezogen auf das Gewicht des anwendungsbereiten Oxidationsfärbemittels.

Weiterhin bevorzugt beträgt die Gesamtmenge an mindestens einem Tensid in der OFV-haltigen Komponente (K1) des erfindungsgemäßen Oxidationsfärbemittels 0,1 - 20 Gew.-%, bevorzugt 0,5 - 10 Gew.-% und besonders bevorzugt 1,5 - 5 Gew.-%, jeweils bezogen auf das Gewicht der Komponente (K1) des erfindungsgemäßen Oxidationsfärbemittels.

Bevorzugte Tenside und Emulgatoren sind ausgewählt aus anionischen, kationischen, zwitterionischen, amphoteren und nichtionischen Tensiden und Emulgatoren sowie deren Mischungen. Diese Stoffe sind nachfolgend beschrieben.

Bevorzugte erfindungsgemäße Oxidationsfärbemittel sind dadurch gekennzeichnet, dass das mindestens eine enthaltene Tensid ausgewählt ist aus nichtionischen Tensiden und anionischen Tensiden sowie aus Mischungen hiervon. Weitere bevorzugte erfindungsgemäße Oxidationsfärbemittel sind dadurch gekennzeichnet, dass ihre OFV-haltige Komponente (K1) mindestens ein Tensid, ausgewählt aus nichtionischen Tensiden und anionischen Tensiden sowie aus Mischungen hiervon, enthält.

Als anionische Tenside eignen sich alle für die Verwendung am menschlichen Körper geeigneten anionischen oberflächenaktiven Stoffe, die eine wasserlöslich machende, anionische Gruppe, beispielsweise eine Carboxylat-, Sulfat-, Sulfonat- oder Phosphat-Gruppe, und eine lipophile Alkylgruppe mit etwa 8 bis 30 C-Atomen, bevorzugt 8 bis 24 C-Atomen im Molekül aufweisen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono, Di- und Trialkanolammoniumsalze mit 2 bis 4 C-Atomen in der Alkanolgruppe, lineare und verzweigte Fettsäuren mit 8 bis 30 C-Atomen (Seifen), polyethoxylierte Ethercarbonsäuren, Acylsarcoside, Acyltauride, Acylisethionate, Sulfobernsteinsäuremono- und -dialkylester und Sulfobernsteinsäuremono-alkylpolyoxyethylester mit 1 bis 6 Ethylenoxidgruppen, lineare Alkansulfonate, lineare alpha-Olefinsulfonate, Sulfonate ungesättigter Fettsäuren mit bis zu 6 Doppelbindungen, alpha-Sulfofettsäuremethylester von Fettsäuren, $C_8$-$C_{20}$-Alkylsulfate und $C_8$-$C_{20}$-Alkylethersulfate mit zu bis 15 Oxyethylgruppen, Gemische oberflächenaktiver Hydroxysulfonate, sulfatierte Hydroxyalkylpolyethylen- und/oder Hydroxyalkylenpropylenglykolether, Ester von Weinsäure oder Zitronensäure mit ethoxylierten oder propoxylierten Fettalkoholen, gegebenenfalls polyethoxylierte Alkyl- und/oder Alkenyletherphosphate, sulfatierte Fettsäurealkylenglykolester, sowie Monoglyceridsulfate und Monoglyceridethersulfate. Bevorzugte anionische Tenside sind Seifen, $C_8$-$C_{20}$-Alkylsulfate, $C_8$-$C_{20}$-Alkylethersulfate und $C_8$-$C_{20}$-Ethercarbonsäuren mit 8 bis 20 C-Atomen in der Alkylgruppe und bis zu 12 Ethylenoxidgruppen im Molekül. Besonders bevorzugt ist Natriumcetearylsulfat.

Bevorzugt beträgt die Gesamtmenge an mindestens einem anionischen Tensid in den erfindungsgemäßen Oxidationsfärbemitteln 0,01 - 10 Gew.-%, bevorzugt 0,1 - 5 Gew.-% und besonders bevorzugt 1 - 3 Gew.-%, jeweils bezogen auf das Gewicht des anwendungsbereiten Oxidationsfärbemittels.

Weiterhin bevorzugt beträgt die Gesamtmenge an mindestens einem anionischen Tensid in der OFV-haltigen Komponente (K1) des erfindungsgemäßen Oxidationsfärbemittels 0,01 - 10 Gew.-%, bevorzugt 0,1 - 5 Gew.-% und besonders bevorzugt 1 - 3 Gew.-%, jeweils bezogen auf das Gewicht der Komponente (K1) des erfindungsgemäßen Oxidationsfärbemittels.

Besonders bevorzugt verwendete nichtionische Tenside sind ausgewählt sind aus mit 20 - 100 Mol Ethylenoxid pro Mol ethoxyliertem Rizinusöl, ethoxylierten $C_8$-$C_{24}$-Alkanolen mit 1 - 200 Mol Ethylenoxid pro Mol, ethoxylierten $C_8$-$C_{24}$-Carbonsäuren mit 1 - 200 Mol Ethylenoxid pro Mol, mit 4 - 80 Mol Ethylenoxid pro Mol ethoxylierten Sorbitanmonoestern von linearen gesättigten und ungesättigten $C_{12}$-$C_{30}$-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, sowie Mischungen der vorgenannten Substanzen.

Die ethoxylierten $C_8$-$C_{24}$-Alkanole haben die Formel $R^1O(CH_2CH_2O)_nH$, wobei $R^1$ steht für einen linearen oder

verzweigten Alkyl- und/oder Alkenylrest mit 8 - 24 Kohlenstoffatomen und n, die mittlere Anzahl der Ethylenoxid-Einheiten pro Molekül, für Zahlen von 1 - 200, bevorzugt 2 - 150, besonders bevorzugt 4 bis 100, außerordentlich bevorzugt 10 - 50, weiter außerordentlich bevorzugt 12 - 30 oder 20 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 1 - 200 Mol Ethylenoxid an technische Fettalkohole mit 12 - 18 Kohlenstoffatomen, wie beispielsweise Kokos-, Palm-, Palmkern- oder Talgfettalkohol, sind geeignet. Besonders bevorzugt sind Laureth-2, Laureth-4, Laureth-10, Laureth-12, Laureth-15, Laureth-20, Laureth-30, Myreth-2, Myreth-4, Myreth-10, Myreth-12, Myreth-15, Myreth-20, Myreth-30, Ceteth-2, Ceteth-4, Ceteth-10, Ceteth-12, Ceteth-15, Ceteth-20, Ceteth-30, Ceteth-50, Ceteth-100, Ceteth-150, Steareth-2, Steareth-4, Steareth-10, Steareth-12 Steareth-15, Steareth-20, Steareth-30, Steareth-50, Steareth-100, Steareth-150, Oleth-2, Oleth-4, Oleth-10, Oleth-12, Oleth-15, Oleth-20, Oleth-30, Ceteareth-2, Ceteareth-4, Ceteareth-10, Ceteareth-15, Ceteareth-12, Ceteareth-15, Ceteareth-20, Ceteareth-30, Ceteareth-50, Ceteareth-100, Ceteareth-150 sowie Coceth-2, Coceth-4, Coceth-10, Coceth-12, Coceth-15, Coceth-20, Coceth-30 Coceth-50 und Coceth-100.

Die ethoxylierten $C_8$-$C_{24}$-Carbonsäuren haben die Formel $R^1O(CH_2CH_2O)_nH$, wobei $R^1O$ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 1 - 200, bevorzugt 10 - 50 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 1 - 200, bevorzugt 10 - 50 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Bevorzugte mit 4 - 80 Mol Ethylenoxid pro Mol ethoxylierte Sorbitanmonoester von linearen gesättigten und ungesättigten $C_{12}$- $C_{30}$-Carbonsäuren, die hydroxyliert sein können, sind ausgewählt aus Polysorbate-20, Polysorbate-40, Polysorbate-60 und Polysorbate-80.

Weitere bevorzugte nichtionische Tenside sind ausgewählt aus $C_8$-$C_{22}$-Alkylmono- und -oligoglycosiden. $C_8$ -$C_{22}$-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, bevorzugt 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene $C_8$-$C_{16}$-Alkylgruppe an einem Oligoglucosidrest, dessen mittlerer Oligomerisationsgrad bei 1 - 2, insbesondere 1,2 - 1,4, liegt. Besonders bevorzugte $C_8$ - $C_{22}$-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die von Glucamin abgeleiteten Acylglucamide sind als nicht-ionische Öl-in-Wasser-Emulgatoren geeignet.

Weitere erfindungsgemäß geeignete nichtionische Tenside enthalten als hydrophile Gruppe z. B. eine Polyolgruppe, eine Polyalkylenglykolethergruppe oder eine Kombination aus Polyol- und Polyglycolethergruppe. Solche Verbindungen sind beispielsweise

- Polyglycerinester und ethoxylierte Polyglycerinester von C8-C30-Fettsäuren, wie beispielsweise Poly(3)glycerindiisostearat (Handelsprodukt: Lameform®TGI (BASF)) und Poly(2)glycerinpoly-hydroxystearat (Handelsprodukt: Dehymuls®PGPH (BASF)),
- ethoxylierte Mono-, Di- und Triester von Glycerin mit C8-C30-Fettsäuren, wie beispielsweise Glycerinmonolaurat + 20 Ethylenoxid und Glycerinmonostearat + 20 Ethylenoxid, PEG-x Castor Oil mit Ethoxylierungsgrad x = 1 - 80 oder PEG-x Hydrogenated Castor Oil mit Ethoxylierungsgrad x = 1 - 80,
- Aminoxide von C8-C30-Fettaminen,
- Zuckerfettsäureester und Anlagerungsprodukte von Ethylenoxid an Zuckerfettsäureester, z. B. Sucrosestearat, Methylglucosesesquistearat, PEG-20-Methylglucosesesquistearat oder PEG-120-Methylglucosedioleat,
- Anlagerungsprodukte von Ethylenoxid an Fettsäurealkanolamide und Fettamine,
- Fettsäure-N-alkylglucamide,
- Monoester von $C_8$-$C_{30}$-Fettsäuren und Ethylenglycol, sowie
- Monoester und Diester von $C_8$-$C_{30}$-Fettsäuren und Glycerin, z. B. Glycerinmonostearat oder Glycerindistearat.

Erfindungsgemäß bevorzugte Oxidationsfärbemittel sind daher dadurch gekennzeichent, dass sie mindestens ein nichtionisches Tensid enthalten, ausgewählt aus mit 20 - 100 Mol Ethylenoxid pro Mol ethoxyliertem Rizinusöl,

ethoxylierten $C_8$-$C_{24}$-Alkanolen mit 1 - 200 Mol Ethylenoxid pro Mol, ethoxylierten $C_8$-$C_{24}$-Carbonsäuren mit 1 - 200 Mol Ethylenoxid pro Mol, mit 4 - 80 Mol Ethylenoxid pro Mol ethoxylierten Sorbitanmonoestern von linearen gesättigten und ungesättigten $C_{12}$- $C_{30}$-Carbonsäuren, die hydroxyliert sein können, insbesondere diejenigen von Myristinsäure, Palmitinsäure, Stearinsäure oder von Mischungen dieser Fettsäuren, Alkylmono- und -oligoglycosiden mit 8 bis 22 Kohlenstoffatomen im Alkylrest und deren ethoxylierten Analoga, von Glucamin abgeleiteten Acylglucamiden, Polyglycerinestern und ethoxylierten Polyglycerinestern von C8-C30-Fettsäuren, ethoxylierten Mono-, Di- und Triestern von Glycerin mit C8-C30-Fettsäuren, Aminoxiden von C8-C30-Fettaminen, Zuckerfettsäureestern und Anlagerungsprodukten von Ethylenoxid an Zuckerfettsäureester, Anlagerungsprodukten von Ethylenoxid an Fettsäurealkanolamide und Fettamine, Fettsäure-N-alkylglucamiden, Monoestern von $C_8$-$C_{30}$-Fettsäuren und Ethylenglycol, Monoestern und Diestern von $C_8$-$C_{30}$-Fettsäuren und Glycerin sowie Mischungen der vorgenannten Substanzen.

Bevorzugt beträgt die Gesamtmenge an mindestens einem nichtionischen Tensid in den erfindungsgemäßen Oxidationsfärbemitteln 0,01 - 15 Gew.-%, bevorzugt 0,1 - 10 Gew.-% und besonders bevorzugt 1 - 6 Gew.-%, jeweils bezogen auf das Gewicht des anwendungsbereiten Oxidationsfärbemittels.

In einer anderen bevorzugten Ausführungsform beträgt die Gesamtmenge an mindestens einem nichtionischen Tensid in der OFV-haltigen Komponente (K1) des erfindungsgemäßen Oxidationsfärbemittels 0,01 - 15 Gew.-%, bevorzugt 0,1 - 10 Gew.-% und besonders bevorzugt 1 - 6 Gew.-%, jeweils bezogen auf das Gewicht der Komponente (K1) des erfindungsgemäßen Oxidationsfärbemittels.

In einer anderen bevorzugten Ausführungsform beträgt die Gesamtmenge an mindestens einem nichtionischen Tensid in der Oxidationsmittel-haltigen Komponente (K2) des erfindungsgemäßen Oxidationsfärbemittels 0,01 - 15 Gew.-%, bevorzugt 0,1 - 10 Gew.-% und besonders bevorzugt 1 - 4 Gew.-%, jeweils bezogen auf das Gewicht der Komponente (K2) des erfindungsgemäßen Oxidationsfärbemittels. In einer anderen bevorzugten Ausführungsform enthält das erfindungsgemäße Oxidationsfärbemittel insgesamt 0,1 bis 15 Gew.-%, bevorzugt 0,5 bis 10 Gew.-% und besonders bevorzugt 1 - 5 Gew.-%, jeweils bezogen auf das Gewicht des anwendungsbereiten Oxidationsfärbemittels, einer Mischung aus nichtionischen und anionischen Tensiden.

In einer anderen bevorzugten Ausführungsform enthält die OFV-haltige Komponente (K1) des erfindungsgemäßen Oxidationsfärbemittels insgesamt 0,1 bis 15 Gew.-%, bevorzugt 0,5 bis 10 Gew.-% und besonders bevorzugt 1 - 5 Gew.-%, jeweils bezogen auf das Gewicht der Komponente (K1), einer Mischung aus nichtionischen und anionischen Tensiden.

In einer anderen bevorzugten Ausführungsform enthält die Oxidationsmittel-haltige Komponente (K2) des erfindungsgemäßen Oxidationsfärbemittels insgesamt 0,1 bis 15 Gew.-%, bevorzugt 0,5 bis 10 Gew.-% und besonders bevorzugt 1 - 5 Gew.-%, jeweils bezogen auf das Gewicht der Komponente (K2), einer Mischung aus nichtionischen und anionischen Tensiden.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder SulfatGruppe tragen. Besonders geeignete zwitterionische Tenside sind die so genannten Betaine wie die N-Alkyl-N,N-dimethylammoniumglycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter amphoteren Tensiden werden solche oberflächenaktiven Verbindungen verstanden, die außer einer $C_8$-$C_{24}$-Alkyl- oder -Acylgruppe im Molekül mindestens eine freie Aminogruppe und mindestens eine -COOH- oder -SOsH-Gruppe enthalten und zur Ausbildung innerer Salze befähigt sind. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte amphotere Tenside sind N-Kokosalkylaminopropionat, Kokosacylaminoethylaminopropionat und $C_{12}$-$C_{18}$-Acylsarcosin.

In einer weiteren bevorzugten Ausführungsform enthält das erfindungsgemäße Oxidationsfärbemittel insgesamt 0,1 bis 10 Gew.-%, bevorzugt 0,2 bis 6 Gew.-% und besonders bevorzugt 1 - 2 Gew.-%, jeweils bezogen auf das Gewicht des anwendungsbereiten Oxidationsfärbemittels, mindestens eines zwitterionischen oder/und eines amphoteren Tensids.

In einer anderen bevorzugten Ausführungsform enthält die OFV-haltige Komponente (K1) des erfindungsgemäßen Oxidationsfärbemittels insgesamt 0,1 bis 10 Gew.-%, bevorzugt 0,2 bis 6 Gew.-% und besonders bevorzugt 1 - 4 Gew.-%, jeweils bezogen auf das Gewicht der Komponente (K1), mindestens eines zwitterionischen oder/und eines amphoteren Tensids.

Optional enthält das erfindungsgemäße Oxidationsfärbemittel, bezogen auf sein Gewicht, mindestens ein kosmetisches Öl in einer Gesamtmenge von 0,01 - 10 Gew.-%, bevorzugt 0,1 - 5 Gew.-%, besonders bevorzugt 0,5 - 4 Gew.-%, außerordentlich bevorzugt 1 - 2 Gew.-%. In einer anderen bevorzugten Ausführungsform enthält die OFV-haltige Komponente (K1) des erfindungsgemäßen Oxidationsfärbemittels insgesamt 0,1 bis 10 Gew.-%, bevorzugt 0,2 bis 6 Gew.-% und besonders bevorzugt 1 - 4 Gew.-%, jeweils bezogen auf das Gewicht der Komponente (K1), mindestens ein kosmetisches Öl. In einer anderen bevorzugten Ausführungsform enthält die Oxidationsmittel-haltige Komponente (K2) des erfindungsgemäßen Oxidationsfärbemittels insgesamt 0,1 bis 10 Gew.-%, bevorzugt 0,2 bis 6 Gew.-% und besonders bevorzugt 1 - 4 Gew.-%, jeweils bezogen auf das Gewicht der Komponente (K2), mindestens ein kosmetisches Öl. Das kosmetische Öl ist unter Normalbedingungen (20 °C, 1013,25 mbar) flüssig; ätherische Öle und Parfümöle bzw. Riechstoffe werden nicht zu den kosmetischen Ölen gezählt. Die unter Normalbedingungen flüssigen kosmetischen Öle sind mit Wasser nicht mischbar. Unter ätherischen Ölen werden erfindungsgemäß Gemische aus flüchtigen Komponenten verstanden, die durch Wasserdampfdestillation aus pflanzlichen Rohstoffen hergestellt werden, wie z. B. Citrusöle. Sofern in der vorliegenden Anmeldung von einem kosmetischen Öl die Rede ist, handelt es sich hierbei immer um ein kosmetisches Öl, das kein Riechstoff und kein ätherisches Öl ist, unter Normalbedingungen flüssig und mit Wasser nicht mischbar ist.

Die Definition eines Riechstoffs im Sinne der vorliegenden Anmeldung stimmt überein mit der fachmännisch üblichen Definition, wie sie dem RÖMPP Chemie Lexikon, Stand Dezember 2007, entnommen werden kann. Danach ist ein Riechstoff eine chemische Verbindung mit Geruch und/ oder Geschmack, der die Rezeptoren der Haarzellen des olfaktorischen Systems erregt (adäquater Reiz). Die hierzu notwendigen physikalischen und chemischen Eigenschaften sind eine niedrige Molmasse von maximal 300 g/mol, ein hoher Dampfdruck, minimale Wasser- und hohe Lipidlöslichkeit sowie schwache Polarität und das Vorliegen mindestens einer osmophoren Gruppe im Molekül. Um flüchtige, niedermolekulare Substanzen, die üblicherweise und auch im Sinne der vorliegenden Anmeldung nicht als Riechstoff, sondern vornehmlich als Lösemittel angesehen und verwendet werden, wie beispielsweise Ethanol, Propanol, Isopropanol und Aceton, von erfindungsgemäßen Riechstoffen abzugrenzen, weisen erfindungsgemäße Riechstoffe eine Molmasse von 74 bis 300 g/mol auf, enthalten mindestens eine osmophore Gruppe im Molekül und weisen einen Geruch und/oder Geschmack auf, das heißt, sie erregen die Rezeptoren der Haarzellen des olfaktorischen Systems.

Erfindungsgemäß besonders bevorzugte Öle sind ausgewählt aus den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Dazu zählen Cetyl-2-ethylhexanoat, 2-Hexyldecylstearat, 2-Hexyldecyllaurat, Isodecylneopentanoat, Isononylisononanoat, 2-Ethylhexylpalmitat und 2-Ethylhexylstearat. Ebenfalls bevorzugt sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und Ethylenglycoldipalmitat.

Weitere erfindungsgemäß bevorzugte Öle sind ausgewählt aus natürlichen und synthetischen Kohlenwasserstoffen, besonders bevorzugt aus Mineralölen, Paraffinölen, $C_{18}$-$C_{30}$-Isoparaffinen, insbesondere Isoeicosan, Polyisobutene und Polydecene, die beispielsweise unter der Bezeichnung Emery® 3004, 3006, 3010 oder unter der Bezeichnung Ethylflo® von Albemarle oder Nexbase® 2004G von Nestle erhältlich sind, weiterhin ausgewählt aus $C_8$-$C_{16}$-Isoparaffinen, insbesondere aus Isodecan, Isododecan, Isotetradecan und Isohexadecan sowie Mischungen hiervon, sowie 1,3-Di-(2-ethylhexyl)-cyclohexan.

Weitere erfindungsgemäß bevorzugte Öle sind ausgewählt aus den Benzoesäureestern von linearen oder verzweigten C8-22-Alkanolen. Besonders bevorzugt sind Benzoesäure-C12-C15-alkylester, Benzoesäureisostearylester, Ethylhexylbenzoat und Benzoesäureoctyldocecylester.

Weitere erfindungsgemäß bevorzugte Öle sind ausgewählt aus Fettalkoholen mit 6 - 30 Kohlenstoffatomen, die ungesättigt oder verzweigt und gesättigt oder verzweigt und ungesättigt sind. Die verzweigten Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol, 2-Ethylhexylalkohol und Isostearylalkohol.

Weitere bevorzugte Öle sind ausgewählt aus Mischungen von Guerbetalkoholen und Guerbetalkoholestern, z.B. Mischungen von 2-Hexyldecanol und 2-Hexyldecyllaurat.

Weitere erfindungsgemäß bevorzugte kosmetische Öle sind ausgewählt aus den Triglyceriden (= Dreifachestern des Glycerins) von linearen oder verzweigten, gesättigten oder ungesättigten, gegebenenfalls hydroxylierten C8-30-Fettsäuren. Besonders bevorzugt kann die Verwendung natürlicher Öle, z.B. Amaranthsamenöl, Aprikosenkernöl, Arganöl, Avocadoöl, Babassuöl, Baumwollsaatöl, Borretschsamenöl, Camelinaöl, Distelöl, Erdnussöl, Granatapfelkernöl, Grapefruitsamenöl, Hanföl, Haselnussöl, Holundersamenöl, Johannesbeersamenöl, Jojobaöl, Leinöl, Macadamianussöl, Maiskeimöl, Mandelöl, Marulaöl, Nachtkerzenöl, Olivenöl, Palmöl, Palmkernöl, Paranussöl, Pekannussöl, Pfirsichkernöl Rapsöl, Rizinusöl, Sanddornfruchtfleischöl, Sanddornkernöl, Sesamöl, Sojaöl, Sonnenblumenöl, Traubenkernöl, Walnussöl, Wildrosenöl, Weizenkeimöl, und die flüssigen Anteile des Kokosöls und dergleichen sein. Bevorzugt sind aber auch synthetische Triglyceridöle, insbesondere Capric/ Caprylic Triglycerides, z. B. die Handelsprodukte Myritol® 318 oder Myritol® 331 (BASF) mit unverzweigten Fettsäureresten sowie Glyceryltriisostearin mit verzweigten Fettsäureresten.

Weitere erfindungsgemäß besonders bevorzugte kosmetische Öle sind ausgewählt aus den Dicarbonsäureestern von linearen oder verzweigten C2-C10-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß bevorzugte kosmetische Öle sind ausgewählt aus den Anlagerungsprodukten von 1 bis 5 Propylenoxid-Einheiten an ein- oder mehrwertige C8-22-Alkanole, wie Octanol, Decanol, Decandiol, Laurylalkohol, Myristylalkohol und Stearylalkohol, z. B. PPG-2-Myristylether und PPG-3-Myristylether.

Weitere erfindungsgemäß bevorzugte kosmetische Öle sind ausgewählt aus den Anlagerungsprodukten von mindestens 6 Ethylenoxid- und/oder Propylenoxid-Einheiten an ein- oder mehrwertige C3-22-Alkanole wie Glycerin, Butanol, Butandiol, Myristylalkohol und Stearylalkohol, die gewünschtenfalls verestert sein können, z. B. PPG-14-Butylether, PPG-9-Butylether, PPG-10-Butandiol, PPG-15-Stearylether und Glycereth-7-diisononanoat.

Weitere erfindungsgemäß bevorzugte kosmetische Öle sind ausgewählt aus den C8-C22-Fettalkoholestern einwertiger oder mehrwertiger C2-C7-Hydroxycarbonsäuren, insbesondere die Ester der Glycolsäure, Milchsäure, Äpfelsäure, Weinsäure, Citronensäure und Salicylsäure.

Weitere erfindungsgemäß bevorzugte kosmetische Öle sind ausgewählt aus den symmetrischen, unsymmetrischen oder cyclischen Estern der Kohlensäure mit $C_{3-22}$-Alkanolen, $C_{3-22}$-Alkandiolen oder $C_{3-22}$-Alkantriolen, z. B. Dicaprylylcarbonat oder die Ester gemäß der Lehre der DE 19756454 A1, insbesondere Glycerincarbonat.

Weitere kosmetische Öle, die erfindungsgemäß bevorzugt sein können, sind ausgewählt aus den Estern von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertigen linearen oder verzweigten $C_2$-$C_6$-Alkanolen.

Weitere kosmetische Öle, die erfindungsgemäß geeignet sind, sind ausgewählt aus den Siliconölen, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Bevorzugt können flüchtige Siliconöle sein, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls geeignet sind flüchtige lineare Siliconöle, insbesondere Hexamethyldisiloxan ($L_2$), Octamethyltrisiloxan ($L_3$), Decamethyltetrasiloxan ($L_4$) sowie beliebige Zweier- und Dreiermischungen aus $L_2$, $L_3$ und/ oder $L_4$, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind. Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5 - 50 cSt oder auch 5 - 10 cSt, und Dimethylpolysiloxan mit einer kinematischen Viskosität (25°C) von etwa 350 cSt.

Es kann erfindungsgemäß außerordentlich bevorzugt sein, Mischungen der vorgenannten Öle einzusetzen.

Bevorzugte erfindungsgemäße Färbemittel sind dadurch gekennzeichnet, dass das kosmetische Öl ausgewählt ist aus natürlichen und synthetischen Kohlenwasserstoffen, besonders bevorzugt aus Paraffinölen, $C_{18}$-$C_{30}$-Isoparaffinen, insbesondere Isoeicosan, Polyisobutene und Polydecene, $C_8$-$C_{16}$-Iso-paraffinen, sowie 1,3-Di-(2-ethylhexyl)-cyclohexan; den Benzoesäureestern von linearen oder verzweigten $C_{8-22}$-Alkanolen; Fettalkoholen mit 6 - 30 Kohlenstoffatomen, die ungesättigt oder verzweigt und gesättigt oder verzweigt und ungesättigt sind; Triglyceriden von linearen oder verzweigten, gesättigten oder ungesättigten, gegebenenfalls hydroxylierten $C_{8-30}$-Fettsäuren, insbesondere natürlichen Ölen; den Dicarbonsäureestern von linearen oder verzweigten $C_2$-$C_{10}$-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige $C_{8-22}$-Alkanole; den Anlagerungsprodukten von mindestens 6 Ethylenoxid- und/oder Propylenoxid-Einheiten an ein- oder mehrwertige $C_{3-22}$-Alkanole; den $C_8$-$C_{22}$-Fettalkoholestern einwertiger oder mehrwertiger $C_2$-$C_7$-Hydroxycarbonsäuren; den symmetrischen, unsymmetrischen oder cyclischen Estern der Kohlensäure mit $C_{3-22}$-Alkanolen, $C_{3-22}$-Alkandiolen oder $C_{3-22}$-Alkantriolen; den Estern von Dimeren ungesättigter $C_{12}$-$C_{22}$-Fettsäuren (Dimerfettsäuren) mit einwertigen linearen, verzweigten oder zyklischen $C_2$-$C_{18}$-Alkanolen oder mit mehrwertigen linearen oder verzweigten $C_2$-$C_6$-Alkanolen; Siliconölen sowie Mischungen der vorgenannten Substanzen.

Zusätzlich können die erfindungsgemäßen Mehrkomponenten-Verpackungseinheiten auch noch ein oder mehrere weitere getrennt konfektionierte Komponenten enthalten. Bei dieser bzw. diesen zusätzlichen getrennt konfektionierten Komponenten kann es sich beispielsweise um ein Vorbehandlungsmittel oder um ein Nachbehandlungsmittel, wie Shampoos oder Conditioner handeln.

Für eine ausreichende Quellung der Keratinfasern ist das anwendungsbereite oxidative Färbemittel bevorzugt auf einen alkalischen pH-Wert eingestellt. Auch die Färbeprozesse auf Keratinfasern laufen üblicherweise im alkalischen Milieu ab. Um die Keratinfasern und auch die Haut so weit wie möglich zu schonen, ist die Einstellung eines zu hohen pH-Wertes jedoch nicht wünschenswert. Daher ist es bevorzugt, wenn der pH-Wert des anwendungsbereiten Mittels

bei einem Wert von 8,0 bis 10,5, weiter bevorzugt von 8,7 bis 10,3, noch weiter bevorzugt von 9,0 bis 10,2 und besonders bevorzugt von 9,2 bis 10,1 liegen. Bei den angegebenen pH-Werten handelt es sich um Werte, die bei einer Temperatur von 22 °C mit einer Glaselektrode gemessen wurden.

Die für die Einstellung des alkalischen pH-Wertes notwendigen Alkalisierungsmittel sind in der Regel zusammen mit dem mindestens einen Oxidationsfarbstoffvorprodukt in der Komponente (K1) enthalten. Die erfindungsgemäß verwendbaren Alkalisierungsmittel können aus der Gruppe, die gebildet wird aus Ammoniak, Alkanolaminen, basischen Aminosäuren, sowie anorganischen Alkalisierungsmitteln wie (Erd-)Alkalimetallhydroxiden, (Erd-)Alkalimetallmetasilikaten, (Erd-)Alkalimetallphosphaten und (Erd-)Alkalimetallhydrogenphosphaten, ausgewählt werden. Bevorzugte anorganische Alkalisierungsmittel sind Natriumhydroxid, Kaliumhydroxid, Natriumsilicat und Natriummetasilicat. Die als Alkalisierungsmittel einsetzbaren Alkanolamine sind bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine sind ausgewählt aus der Gruppe 2-Aminoethan-1-ol, 2-Amino-2-methylpropan-1-ol und 2-Amino-2-methyl-propan-1,3-diol. Die als erfindungsgemäßes Alkalisierungsmittel einsetzbaren basischen Aminosäuren werden bevorzugt ausgewählt aus der Gruppe, die gebildet wird aus Arginin, Lysin, Ornithin und Histidin, besonders bevorzugt Arginin. Es hat sich aber im Rahmen der Untersuchungen zur vorliegenden Erfindung herausgestellt, dass weiterhin erfindungsgemäß bevorzugte Mittel dadurch gekennzeichnet sind, dass sie zusätzlich ein organisches Alkalisierungsmittel enthalten. Eine Ausführungsform des ersten Erfindungsgegenstands ist dadurch gekennzeichnet, dass das Mittel zusätzlich mindestens ein Alkalisierungsmittel enthält, das ausgewählt ist aus der Gruppe, die gebildet wird aus Ammoniak, Alkanolaminen und basischen Aminosäuren, insbesondere aus Ammoniak, Monoethanolamin und Arginin oder seinen verträglichen Salzen. Bevorzugt sind das oder die Alkalisierungsmittel zusammen mit den Oxidationsfarbstoffvorprodukten in der Färbezubereitung (K1) enthalten.

Bei der zweiten Komponente (K2) des ersten, zweiten, dritten und vierten Gegenstands der Erfindung handelt es sich um eine Oxidationsmittelzubereitung, die Wasserstoffperoxid in wässriger Lösung enthält. Die Konzentration des Wasserstoffperoxids in der Oxidationsmittelzubereitung (K2) wird einerseits von den gesetzlichen Vorgaben und andererseits von dem gewünschten Effekt bestimmt; vorzugsweise werden 6 bis 12 Gew.-%ige Lösungen in Wasser verwendet. Erfindungsgemäß bevorzugte Zubereitungen (K2) sind dadurch gekennzeichnet, dass sie 1 bis 24 Gew.-%, vorzugsweise 3 bis 12,5 Gew.-%, besonders bevorzugt 6 bis 10 Gew.-% und insbesondere 3 bis 6 Gew.-% Wasserstoffperoxid, jeweils bezogen auf das Gewicht der Oxidationsmittelzubereitung (K2), enthalten. Die gewichtsbezogenen Mischverhältnisse von (K1) zu (K2) sind entsprechend so zu wählen, dass das anwendungsbereite Färbemittel, bezogen auf sein Gewicht, 0,5 bis 12 Gew.-%, bevorzugt 0,9 bis 7 Gew.-%, besonders bevorzugt 1,5 bis 5 Gew.-%, außerordentlich bevorzugt 3 bis 4,5 Gew.-% Wasserstoffperoxid (berechnet als 100 %-iges $H_2O_2$) enthält.

Bezüglich weiterer bevorzugter Ausführungsformen des erfindungsgemäßen Mehrkomponenten-Verpackungseinheit (Kit-of-parts) gilt mutatis mutandis das zu den erfindungsgemäßen Mitteln Gesagte.

Zur weiteren Fein-Nuancierung der gewünschten Rotnuance können die erfindungsgemäßen Mittel zusätzlich noch einen oder mehrere weitere Oxidationsfarbstoffe vom Entwicklertyp enthalten. Insbesondere, wenn zusätzlich zu dem mindestens einen Oxidationsfarbstoffvorprodukt (A) noch mindestens eine Verbindung aus der Gruppe, bestehend aus p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, und/oder der physiologisch verträglichen Salze dieser Verbindungen, enthalten ist, konnten gute Ergebnisse erhalten werden. Besonders bevorzugt aus dieser Gruppe ist die Entwicklerkomponente 4-Amino-3-methylphenol, auch als "Oxyrot" bezeichnet, enthalten.

In einer weiteren besonders bevorzugten Ausführungsform ist ein erfindungsgemäßes Mittel dadurch gekennzeichnet, dass es zusätzlich eine oder mehrere Verbindungen aus der Gruppe, bestehend aus p-Aminophenol, 4-Amino-3-methylphenol, 4-Amino-2-aminomethylphenol, 4-Amino-2-(1,2-dihydroxy-ethyl)phenol, 4-Amino-2-(diethylaminomethyl)phenol, und/oder der physiologisch verträglichen Salze dieser Verbindungen, enthält, bevorzugt in einer Gesamtmenge von 0,001 bis 0,4 Gew.-%, besonders bevorzugt 0,01 bis 0,2 Gew.-%, außerordentlich bevorzugt 0,05 bis 0,1 Gew.-%, jeweils bezogen auf das erfindungsgemäße anwendungsbereite Färbemittel.

In einer weiteren besonders bevorzugten Ausführungsform ist ein erfindungsgemäßes Mittel dadurch gekennzeichnet, dass es zusätzlich 4-Amino-3-methylphenol und/oder ein physiologisch verträgliches Salz dieser Verbindung enthält, bevorzugt in einer Gesamtmenge von 0,001 bis 0,4 Gew.-%, besonders bevorzugt 0,01 bis 0,2 Gew.-%, außerordentlich bevorzugt 0,05 bis 0,1 Gew.-%, jeweils bezogen auf das erfindungsgemäße anwendungsbereite Färbemittel.

In einer weiteren besonders bevorzugten Ausführungsform ist ein erfindungsgemäßes Kit dadurch gekennzeichnet, dass die OFV-haltige Komponente (K1) zusätzlich eine oder mehrere Verbindungen aus der Gruppe, bestehend aus p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, und/oder der physiologisch verträglichen Salze dieser Verbindungen, enthält, bevorzugt in einer Gesamtmenge von 0,002 bis 0,8 Gew.-%, besonders bevorzugt 0,02 bis 0,4 Gew.-%, außerordentlich

bevorzugt 0,1 bis 0,2 Gew.-%, jeweils bezogen auf das Gewicht der Komponente (K1).

In einer weiteren besonders bevorzugten Ausführungsform ist ein erfindungsgemäßes Kit dadurch gekennzeichnet, dass die OFV-haltige Komponente (K1) zusätzlich 4-Amino-3-methylphenol und/oder ein physiologisch verträgliches Salz dieser Verbindung enthält, bevorzugt in einer Gesamtmenge von 0,002 bis 0,8 Gew.-%, besonders bevorzugt 0,02 bis 0,4 Gew.-%, außerordentlich bevorzugt 0,1 bis 0,2 Gew.-%, jeweils bezogen auf das Gewicht der Komponente (K1).

Besonders bevorzugt ist weiterhin ein Mittel zum oxidativen Färben von keratinischen Fasern, enthaltend in einem kosmetischen Träger - bezogen auf das Gesamtgewicht des anwendungsbereiten Mittels -

(A) 4,5-Diamino-1-(2-hydroxyethyl)-1H-pyrazol (Struktur I-A), in einer Gesamtmenge von 0,01 bis 2,5 Gew.-%, bevorzugt 0,1 bis 1,8 Gew.-%, besonders bevorzugt 0,2 bis 1,0 Gew.-%, außerordentlich bevorzugt 0,4 bis 0,9 Gew.-%, enthalten ist, wobei sich die Mengenangaben auf das Gewicht der freien 4,5-Diaminopyrazol-Base in Relation zum Gewicht des erfindungsgemäßen Mittels beziehen, weiterhin
(B) Glucoheptonsäure und/oder mindestens eines ihrer physiologisch verträglichen Salze und/oder Lactone, in einer Gesamtmenge von 0,01 bis 2,5 Gew.-%, bevorzugt 0,05 bis 1,5 Gew.-%, besonders bevorzugt 0,1 bis 1,0 Gew.-%, außerordentlich bevorzugt 0,1 bis 0,5 Gew.-% enthalten ist/sind, wobei sich die Mengenangaben auf das Gewicht an freier Glucoheptonsäure in Relation zum Gewicht des erfindungsgemäßen Mittels beziehen, weiterhin
(C) mindestens ein Oxidationsmittel, das von Luftsauerstoff verschieden ist, und

zusätzlich eine oder mehrere Verbindungen aus der Gruppe, bestehend aus p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, und/oder der physiologisch verträglichen Salze dieser Verbindungen, in einer Gesamtmenge von 0,001 bis 0,4 Gew.-%, besonders bevorzugt 0,01 bis 0,2 Gew.-%, außerordentlich bevorzugt 0,05 bis 0,1 Gew.-%, jeweils bezogen auf das erfindungsgemäße anwendungsbereite Färbemittel.

Besonders bevorzugt ist ein Mittel zum oxidativen Färben von keratinischen Fasern, enthaltend in einem kosmetischen Träger - bezogen auf das Gesamtgewicht des anwendungsbereiten Mittels -

(A) 4,5-Diamino-1-(2-hydroxyethyl)-1H-pyrazol (Struktur I-A), in einer Gesamtmenge von 0,01 bis 2,5 Gew.-%, bevorzugt 0,1 bis 1,8 Gew.-%, besonders bevorzugt 0,2 bis 1,0 Gew.-%, außerordentlich bevorzugt 0,4 bis 0,9 Gew.-%, enthalten ist, wobei sich die Mengenangaben auf das Gewicht der freien 4,5-Diaminopyrazol-Base in Relation zum Gewicht des erfindungsgemäßen Mittels beziehen, weiterhin
(B) Natriumglucoheptonat in einer Gesamtmenge von 0,01 bis 2,5 Gew.-%, bevorzugt 0,05 bis 1,5 Gew.-%, besonders bevorzugt 0,1 bis 1,0 Gew.-%, außerordentlich bevorzugt 0,1 bis 0,5 Gew.-% enthalten ist/sind, wobei sich die Mengenangaben auf das Gewicht an freier Glucoheptonsäure in Relation zum Gewicht des erfindungsgemäßen Mittels beziehen, weiterhin
(C) mindestens ein Oxidationsmittel, das von Luftsauerstoff verschieden ist, und

zusätzlich eine oder mehrere Verbindungen aus der Gruppe, bestehend aus p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, und/oder der physiologisch verträglichen Salze dieser Verbindungen, in einer Gesamtmenge von 0,001 bis 0,4 Gew.-%, besonders bevorzugt 0,01 bis 0,2 Gew.-%, außerordentlich bevorzugt 0,05 bis 0,1 Gew.-%, jeweils bezogen auf das erfindungsgemäße anwendungsbereite Färbemittel.

Besonders bevorzugt ist weiterhin ein Mittel zum oxidativen Färben von keratinischen Fasern, enthaltend in einem kosmetischen Träger - bezogen auf das Gesamtgewicht des anwendungsbereiten Mittels -

(A) 4,5-Diamino-1-(2-hydroxyethyl)-1H-pyrazol (Struktur I-A), in einer Gesamtmenge von 0,01 bis 2,5 Gew.-%, bevorzugt 0,1 bis 1,8 Gew.-%, besonders bevorzugt 0,2 bis 1,0 Gew.-%, außerordentlich bevorzugt 0,4 bis 0,9 Gew.-%, enthalten ist, wobei sich die Mengenangaben auf das Gewicht der freien 4,5-Diaminopyrazol-Base in Relation zum Gewicht des erfindungsgemäßen Mittels beziehen, weiterhin
(B) Natriumglucoheptonat in einer Gesamtmenge von 0,01 bis 2,5 Gew.-%, bevorzugt 0,05 bis 1,5 Gew.-%, besonders bevorzugt 0,1 bis 1,0 Gew.-%, außerordentlich bevorzugt 0,1 bis 0,5 Gew.-% enthalten ist/sind, wobei sich die Mengenangaben auf das Gewicht an freier Glucoheptonsäure in Relation zum Gewicht des erfindungsgemäßen Mittels beziehen, weiterhin
(C) mindestens ein Oxidationsmittel, das von Luftsauerstoff verschieden ist, und

zusätzlich 4-Amino-3-methylphenol und/oder der physiologisch verträglichen Salze dieser Verbindung, in einer Gesamtmenge von 0,001 bis 0,4 Gew.-%, besonders bevorzugt 0,01 bis 0,2 Gew.-%, außerordentlich bevorzugt 0,05 bis 0,1 Gew.-%, jeweils bezogen auf das erfindungsgemäße anwendungsbereite Färbemittel.

Weitere erfindungsgemäß bevorzugte Färbemittel sind dadurch gekennzeichnet, dass zusätzliche Oxidationsfarbstoffvorprodukte vom Entwickler-Typ, ausgewählt aus der Gruppe, die gebildet wird aus p-Phenylendiamin, Toluen-2,5-diamin, 2-(2,5-Diaminophenyl)ethanol, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophen-oxy)propan-2-ol, N,N'-Bis-(4-aminophenyl)-1,4-diazacycloheptan, 1,10-Bis-(2,5-diaminophenyl)-1,4,7,10-tetraoxadecan, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo-[1,2-a]-pyrazol-1-on sowie deren physiologisch verträglichen Salzen, nicht oder nur in einer Gesamtmenge von maximal 0,08 Gew.-%, bevorzugt maximal 0,02 Gew.-%, jeweils bezogen auf das Gewicht des anwendungsbereiten erfindungsgemäßen Färbemittels, enthalten sind.

Bevorzugte physiologisch verträgliche Salze der Oxidationsfarbstoffvorprodukte, die eine oder mehrere Amingruppen aufweisen, sind insbesondere die Hydrochloride (Monohydrochlorid × HCl, oder Dihydrochlorid × 2 HCl), das Sulfat (x $H_2SO_4$), und die Hydrobromide (Monohydrobromid × HBr, oder Dihydrobromid × 2 HBr) der Verbindung.

Weiterhin können die erfindungsgemäßen Mittel auch zusätzlich auch noch ein oder mehrere Oxidationsfarbstoffvorprodukte vom Kupplertyp enthalten. Kupplerkomponenten bilden im Rahmen der oxidativen Färbung allein keine signifikante Färbung aus, sondern benötigen stets die Gegenwart von Entwicklerkomponenten. Kupplerkomponenten im Sinne der Erfindung erlauben mindestens eine Substitution eines chemischen Restes des Kupplers durch die oxidierte Form der Entwicklerkomponente. Dabei bilden sich kovalente Bindungen zwischen Kuppler- und Entwicklerkomponente aus.

Als erfindungsgemäß geeignete Kupplerkomponente wird bevorzugt mindestens eine Verbindung aus einer der folgenden Klassen ausgewählt:

- m-Aminophenol und/oder dessen Derivate,
- m-Dihydroxybenzol und/oder dessen Derivate,
- m-Diaminobenzol und/oder dessen Derivate,
- o-Diaminobenzol und/oder dessen Derivate,
- o-Aminophenolderivate, wie beispielsweise o-Aminophenol,
- Naphthalinderivate mit mindestens einer Hydroxygruppe,
- Di- beziehungsweise Trihydroxybenzol und/oder deren Derivate,
- Pyridinderivate,
- Pyrimidinderivate,
- Monohydroxyindol-Derivate und/oder Monoaminoindol-Derivate,
- Monohydroxyindolin-Derivate und/oder Monoaminoindolin-Derivate,
- Pyrazolonderivate, wie beispielsweise 1-Phenyl-3-methylpyrazol-5-on,
- Morpholinderivate, wie beispielsweise 6-Hydroxybenzomorpholin oder 6-Aminobenzomorpholin,
- Chinoxalinderivate, wie beispielsweise 6-Methyl-1,2,3,4-tetrahydrochinoxalin.

Gemische aus zwei oder mehreren Verbindungen aus einer oder mehreren dieser Klassen sind im Rahmen dieser Ausführungsform ebenso erfindungsgemäß.

Eine weitere bevorzugte Ausführungsform ist ein erfindungsgemäßes Mittel, das dadurch gekennzeichnet ist, dass es zusätzlich mindestens ein Oxidationsfarbstoffvorprodukt vom Kuppler-Typ enthält, das ausgewählt ist aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophen-oxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethyl-pyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin und/oder 7-Hydroxyindolin und deren physiologisch verträglichen Salzen.

Prinzipiell können die erfindungsgemäßen Mittel ebenfalls mindestens einen direktziehenden Farbstoff aus der Gruppe der anionischen, nichtionischen und/oder kationischen Farbstoffe enthalten.

Besonders bevorzugt handelt es sich hierbei um einen oder mehrere nichtionische direktziehende Farbstoffe aus der Gruppe, HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue

12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)-amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

In einer weiteren besonders bevorzugten Ausführungsform ist erfindungsgeäßes Mittel dadurch gekennzeichnet, dass es zusätzlich einen oder mehrere nichtionische direktziehende Farbstoffe aus der Gruppe HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydro-chinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol enthält.

Zusätzlich können auch anionische direktziehende Farbstoffe enthalten sein, die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten sind.

Geeigenete kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls geeignete kationische direktziehende Farbstoffe.

Die zusätzlichen Oxidationsfarbstoffvorprodukte, d.h. Entwicklerkomponenten, die von den Verbindungen der Gruppe (A) verschieden sind, weiterhin Kupplerkomponenten sowie die optional zusätzlich enthaltenen direktziehenden Farbstoffe können beispielsweise in einer Menge von 0,0001 bis 5,0 Gew.-%, vorzugsweise 0,001 bis 0,5 Gew.-%, jeweils bezogen auf das Gesamtgewicht des erfindungsgemäßen Mittels, enthalten sein.

Ferner können die erfindungsgemäßen Mittel weitere Wirk-, Hilfs- und Zusatzstoffe enthalten, wie beispielsweise nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/ oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente

sowie Treibmittel wie Propan-Butan-Gemische, NzO, Dimethylether, COz und Luft.

Ein weiterer Gegenstand der vorliegenden Erfindung ist ein Verfahren zur oxidativen Haarfärbung von keratinischen Fasern, insbesondere menschlichen Haaren, bei dem ein erfindungsgemäßes oder erfindungsgemäß bevorzugtes Mittel, insbesondere ein Mittel gemäß einem der Ansprüche 1 bis 4, auf die Fasern, insbesondere die Haare, aufgetragen wird, dort für eine Zeit von 1 bis 60 Minuten, bevorzugt von 20 bis 45 Minuten, bei Raumtemperatur und/oder bei mindestens 30 °C auf den Fasern verbleibt, die Fasern, insbesondere die Haare, anschließend mit Wasser und/oder einer reinigenden Zusammensetzung gespült werden und gegebenenfalls ein Nachbehandlungsmittel auf die Fasern, insbesondere die Haare, appliziert wird, das gegebenenfalls ausgespült wird, und die Fasern, insbesondere die Haare, anschließend getrocknet werden.

Ein weiterer Gegenstand der vorliegenden Erfindung ist ein Verfahren zur oxidativen Haarfärbung von keratinischen Fasern, insbesondere menschlichen Haaren, bei dem die Komponenten eines erfindungsgemäßen oder erfindungsgemäß bevorzugten Kits, insbesondere eines Kits gemäß einem der Ansprüche 5 und 6 , gleichzeitig oder direkt nacheinander ohne Ausspülen auf die Fasern, insbesondere die Haare, aufgetragen werden, dort für eine Zeit von 1 bis 60 Minuten, bevorzugt von 20 bis 45 Minuten, bei Raumtemperatur und/oder bei mindestens 30 °C auf den Fasern verbleiben, die Fasern, insbesondere die Haare, anschließend mit Wasser und/oder einer reinigenden Zusammensetzung gespült werden und gegebenenfalls ein Nachbehandlungsmittel auf die Fasern, insbesondere die Haare, appliziert wird, das gegebenenfalls ausgespült wird, und die Fasern, insbesondere die Haare, anschließend getrocknet werden.

Der Begriff "Raumtemperatur" bezeichnet erfindungsgemäß die Temperatur in dem Raum, in dem eine Person üblicherweise ein Haarfärbemittel benutzt, also üblicherweise ein Badezimmer oder ein Friseursalon, in dem eine Temperatur im Bereich von 10 - 29 °C herrscht.

Das Belassen der Haar färbenden Anwendungsmischung auf den Fasern, insbesondere den Haaren, kann auch bei mindestens 30 °C, bevorzugt bei 30 - 60°C, besonders bevorzugt bei 32 - 50°C erfolgen, wenn das Haar beispielsweise mit einer Wärmehaube oder mit einem Wärmestrahler erwärmt wird. Die in erfindungsgemäßen und erfindungsgemäß bevorzugten Färbekits sowie in erfindungsgemäßen und erfindungsgemäß bevorzugten Färbeverfahren eingesetzte Oxidationsmittelzubereitung (K2) enthält, jeweils bezogen auf ihr Gewicht, bevorzugt 40 - 96 Gew.-%, besonders bevorzugt 70 - 93 Gew.-%, außerordentlich bevorzugt 80 - 90 Gew.-%, Wasser.

Die in erfindungsgemäßen und erfindungsgemäß bevorzugten Färbekits sowie in erfindungsgemäßen und erfindungsgemäß bevorzugten Färbeverfahren eingesetzte Oxidationsmittelzubereitung (K2) enthält weiterhin, jeweils bezogen auf ihr Gewicht, bevorzugt 0,5 bis 23 Gew.-%, weiter bevorzugt 2,5 bis 21 Gew.-%, besonders bevorzugt 4 bis 20 Gew.-%, ganz besonders bevorzugt 5 bis 18 Gew.-% und außerordentlich bevorzugt 6 bis 12 Gew.-%, Wasserstoffperoxid.

Zur Stabilisierung des Wasserstoffperoxids weist die Oxidationsmittelzubereitung (K2) bevorzugt einen pH-Wert im Bereich von 2,0 bis 6,5, besonders bevorzugt 2,5 - 5,5, außerordentlich bevorzugt 2,8 bis 5,0, auf, jeweils gemessen bei 20°C.

Kationtensid in der Oxidationsmittelzubereitung (K2)

Die Oxidationsmittelzubereitung (K2) weist üblicherweise eine Viskosität im Bereich von 10 - 6000 mPas, bevorzugt 200 - 5000 mPas, besonders bevorzugt 1000 - 4500 mPas, auf, jeweils gemessen bei 20°C. Für die Applikation auf das Haar allerdings sollte die Anwendungsmischung eine deutlich höhere Viskosität aufweisen, damit sie während der gesamten Einwirkzeit (im Bereich von 5 - 60 Minuten, bevorzugt 30 - 45 Minuten) auf dem Haar verbleibt und nicht heruntertropft. Hierbei unterscheidet man, ob die Anwendungsmischung durch das Schütteln beider Zusammensetzungen (K1) und (K2) in einer Applikationsflasche hergestellt wird, aus der heraus die Anwendungsmischung unverzüglich nach dem Mischen mit Hilfe einer Applikationstülle als Flaschenaufsatz auf das Haar appliziert wird (Flaschenapplikation), oder ob die Anwendungsmischung durch Verrühren beider Zusammensetzungen (K1) und (K2) in einer Schale hergestellt wird, aus der heraus die Anwendungsmischung unverzüglich nach dem Mischen mit einer Pinsel auf das Haar appliziert wird (Pinselapplikation). Die Flaschenapplikation ist insbesondere für Färbemittel geeignet, die im Einzelhandel mit einer Empfehlung zur Anwendung durch den Verbraucher selbst vertrieben werden. Die Pinselapplikation ist insbesondere für Färbemittel geeignet, die im Friseursalon durch den Friseur hergestellt und auf die Haare des Verbrauchers appliziert werden.

Überraschenderweise wurde gefunden, dass man eine Anwendungsmischung mit einer insbesondere für die Pinselapplikation geeigneten Viskosität erhält, wenn man eine erfindungsgemäße oder erfindungsgemäß bevorzugte Komponente (K1) mit einer Oxidationsmittelzubereitung (K2) vermischt, die mindestens ein Kationtensid enthält, insbesondere, wenn (K1) ein Aniontensid enthält. Beim Vermischen führt die Wechselwirkung zwischen anionischen Bestandteilen in (K1) und dem mindestens einen Kationtensid zum gewünschten Anstieg der Viskosität. Die damit erzielte pastöse Konsistenz der Anwendungsmischung führt zu optimalen Anwendungseigenschaften, insbesondere für die Pinselapplikation. Die so erzielten Anwendungsmischungen, insbesondere die Mischungen, deren Gewichts-bezogenes Mischungsverhältnis (K1):(K2) im Bereich von 1:0,8 bis 1:2,5, besonders bevorzugt im Bereich von 1:1 bis 1:2 liegt,

weisen bevorzugt eine Viskosität im Bereich von 20000 - 100000 mPas, bevorzugt 30000 - 80000 mPas, besonders bevorzugt 45000 - 70000 mPas, auf, jeweils gemessen bei 20°C (Brookfield-Viskosimeter, Rotationsfrequenz von 4 $min^{-1}$, Spindel Nr. 5).

In einer weiteren bevorzugten Ausführungsform der Erfindung enthält die erfindungsgemäß eingesetzte Oxidationsmittelzubereitung (K2) mindestens ein Kationtensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2).

Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Kationische Tenside adsorbieren an Grenzflächen und aggregieren in wässriger Lösung oberhalb der kritischen Mizellbildungskonzentration zu positiv geladenen Mizellen.

Erfindungsgemäß bevorzugt sind kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride, Trialkylmethylammoniumchloride, sowie die unter den INCI-Bezeichnungen Quaternium-27 und Quaternium-83 bekannten Imidazolium-Verbindungen. Weitere bevorzugte quaternäre Ammoniumverbindungen sind Tetraalkylammoniumsalze, wie insbesondere das unter der INCI-Bezeichnung bekannte Quaternium-52, ein Poly(Oxy-1,2-Ethanediyl), ((Octadecylnitrilio)tri-2,1-Ethanediyl)tris(Hydroxy)-Phosphat (1:1)-Salz, das die allgemeine Strukturformel (III) aufweist, worin x + y + z = 10 sind:

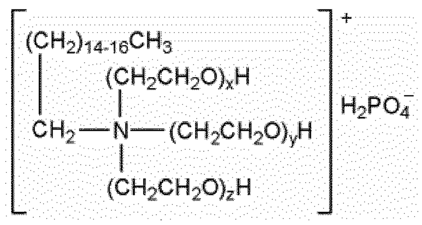

(III).

Die langen Alkylketten der oben genannten Tenside weisen bevorzugt 10 bis 22, besonders bevorzugt 12 bis 18 Kohlenstoffatome auf. Besonders bevorzugt sind Behenyltrimethylammoniumchlorid, Stearyltrimethylammoniumchlorid und Cetyltrimethylammoniumchlorid, wobei Stearyltrimethylammoniumchlorid außerordentlich bevorzugt ist. Weitere erfindungsgemäß geeignete kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt. Eine erfindungsgemäß geeignete Verbindung aus dieser Substanzgruppe stellt Tegoamid® S 18 (Stearamidopropyldimethylamin) dar. Bei Esterquats handelt es sich um Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden unter den Warenzeichen Stepantex, Dehyquart und Armocare vertrieben.

In Bezug auf optimale Anwendungseigenschaften und optimale Färbeergebnisse haben sich C10-C22-Alkyltrimethylammoniumchloride als besonders gut geeignet herausgestellt. Besonders bevorzugte erfindungsgemäß verwendete Oxidationsmittelzubereitungen (K2) sind daher dadurch gekennzeichnet, dass sie mindestens ein kationisches Tensid in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2), enthalten, wobei bevorzugt mindestens ein Tensid, ausgewählt aus C10-C22-Alkyltrimethylammoniumchloriden, insbesondere ausgewählt aus Behenyltrimethylammoniumchlorid, Stearyltrimethylammoniumchlorid und Cetyltrimethylammoniumchlorid sowie Mischungen dieser Tenside, enthalten sind. Außerordentlich bevorzugte erfindungsgemäß verwendete Oxidationsmittelzubereitungen (K2) enthalten Stearyltrimethylammoniumchlorid in einer Gesamtmenge von 0,05 - 3 Gew.-%, besonders bevorzugt von 0,1 - 1,5 Gew.-%, außerordentlich bevorzugt 0,3 - 0,9 Gew.-%, jeweils bezogen auf das Gewicht der Oxidationsmittelzubereitung (K2).

Eine weitere erfindungsgemäß bevorzugte Verpackungseinheit (Kit-of-Parts) ist dadurch gekennzeichnet, dass die Oxidationsmittelzubereitung (K2) mindestens ein kationisches Tensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher enthält.

Eine weitere erfindungsgemäß bevorzugte Verpackungseinheit (Kit-of-Parts) ist dadurch gekennzeichnet, dass die Oxidationsmittelzubereitung (K2) mindestens ein kationisches Tensid, das bevorzugt ausgewählt ist aus Stearyltrimethylammoniumchlorid, in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher enthält.

Ein erfindungsgemäß bevorzugtes Verfahren zur oxidativen Haarfärbung ist dadurch gekennzeichnet, dass die Oxidationsmittelzubereitung (K2) mindestens ein kationisches Tensid, bevorzugt in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher enthält.

Ein weiteres erfindungsgemäß bevorzugtes Verfahren zur oxidativen Haarfärbung ist dadurch gekennzeichnet, dass die Oxidationsmittelzubereitung (K2) mindestens ein kationisches Tensid, das bevorzugt ausgewählt ist aus Stearyltrimethylammoniumchlorid, in einer Gesamtmenge von 0,05 - 3 Gew.-%, besonders bevorzugt von 0,1 - 1,5 Gew.-%, außerordentlich bevorzugt von 0,3 - 0,9 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher enthält.

Überraschenderweise wurde gefunden, dass man eine Anwendungsmischung mit einer insbesondere für die Flaschenapplikation geeigneten Viskosität erhält, wenn man eine erfindungsgemäße oder erfindungsgemäß bevorzugte Komponente (K1) mit einer Oxidationsmittelzubereitung (K2) vermischt, die mindestens ein Copolymer, ausgewählt aus vernetzten Acrylsäure/Acrylsäure-C1-C6-Alkylester-Copolymeren und vernetzten Methacrylsäure/Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (K2), enthält. Das Vermischen des erfindungsgemäßen oder erfindungsgemäß bevorzugten Mittels mit einer solchen Oxidationsmittelzubereitung (K2) führt zum gewünschten Anstieg der Viskosität. Die damit erzielte mittelviskose Konsistenz der Anwendungsmischung führt zu optimalen Anwendungseigenschaften, insbesondere für die Flaschenapplikation. Die so erzielten Anwendungsmischungen, insbesondere bei Gewichts-bezogenen Mischungsverhältnissen (K1):(K2) im Bereich von 1:0,8 bis 1:2,5, besonders bevorzugt im Bereich von 1:1 bis 1:2, weisen bevorzugt eine Viskosität im Bereich von 10000 - 50000 mPas, bevorzugt 15000 - 30000 mPas, besonders bevorzugt 18000 - 25000 mPas, auf, jeweils gemessen bei 20°C (Brookfield-Viskosimeter, Rotationsfrequenz 4 $min^{-1}$, Spindel Nr. 5).

Eine weitere erfindungsgemäß bevorzugte Verpackungseinheit (Kit-of-Parts) ist daher dadurch gekennzeichnet, dass die Oxidationsmittelzubereitung (K2) mindestens ein Copolymer, ausgewählt aus vernetzten Acrylsäure/ Acrylsäure-C1-C6-Alkylester-Copolymeren und vernetzten Methacrylsäure/ Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (K2), und bevorzugt kein Kationtensid enthält.

Ein weiteres erfindungsgemäß bevorzugtes Verfahren zur oxidativen Haarfärbung ist daher dadurch gekennzeichnet, dass die Oxidationsmittelzubereitung (K2) mindestens ein Copolymer, ausgewählt aus vernetzten Acrylsäure/ Acrylsäure-C1-C6-Alkylester-Copolymeren und vernetzten Methacrylsäure/ Acrylsäure-C1-C6-Alkylester-Copolymeren, enthält, bevorzugt in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (K2), und bevorzugt kein Kationtensid enthält.

Bevorzugte vernetzte Copolymere dieser Art sind ausgewählt aus -jeweils vernetzten - Methacrylsäure/ Methylacrylat-, Methacrylsäure/Ethylacrylat-, Methacrylsäure/Propylacrylat-, Methacrylsäure/ Butylacrylat-, Methacrylsäure/Pentylacrylat-, Methacrylsäure/Hexylacrylat-, Acrylsäure/Methylacrylat-, Acrylsäure/Ethylacrylat-, Acrylsäure/Propylacrylat-, Acrylsäure/Butylacrylat-, Acrylsäure/Pentylacrylat- und Acrylsäure/Hexylacrylat- Copolymeren und Mischungen davon.

Eine weitere erfindungsgemäß bevorzugte Verpackungseinheit (Kit-of-Parts) ist dadurch gekennzeichnet, dass die Oxidationsmittelzubereitung (K2) mindestens ein vernetztes Copolymer, ausgewählt aus - jeweils vernetzten - Methacrylsäure/Methylacrylat-, Methacrylsäure/Ethylacrylat-, Methacrylsäure/ Propylacrylat-, Methacrylsäure/ Butylacrylat-, Methacrylsäure/ Pentylacrylat-, Methacrylsäure/ Hexylacrylat-, Acrylsäure/Methylacrylat-, Acrylsäure/Ethylacrylat-, Acrylsäure/Propylacrylat-, Acrylsäure/ Butylacrylat-, Acrylsäure/Pentylacrylat- und Acrylsäure/Hexylacrylat- Copolymeren und Mischungen davon, in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (K2), und kein Kationtensid enthält.

Ein weiteres erfindungsgemäß bevorzugtes Verfahren zur oxidativen Haarfärbung ist dadurch gekennzeichnet, dass die Oxidationsmittelzubereitung (K2) mindestens ein vernetztes Copolymer, ausgewählt aus - jeweils vernetzten - Methacrylsäure/Methylacrylat-, Methacrylsäure/Ethylacrylat-, Methacrylsäure/ Propylacrylat-, Methacrylsäure/ Butylacrylat-, Methacrylsäure/ Pentylacrylat-, Methacrylsäure/ Hexylacrylat-, Acrylsäure/Methylacrylat-, Acrylsäure/Ethylacrylat-, Acrylsäure/Propylacrylat-, Acrylsäure/ Butylacrylat-, Acrylsäure/Pentylacrylat- und Acrylsäure/Hexylacrylat-Copolymeren und Mischungen davon, in einer Gesamtmenge von 0,1 - 7 Gew.-%, besonders bevorzugt 0,5 - 6 Gew.-%, außerordentlich bevorzugt von 1 - 4,5 Gew.-%, jeweils bezogen auf das Gewicht der Oxidationsmittelzubereitung (K2), und kein Kationtensid enthält.

Die erfindungsgemäß verwendeten und erfindungsgemäß bevorzugt verwendeten Oxidationsmittelzubereitungen (K2) können darüber hinaus Stabilisatoren, insbesondere Komplexbildner, und pH-Puffersubstanzen, enthalten.

In einer weiteren bevorzugten Ausführungsform der Erfindung enthält die erfindungsgemäß eingesetzte Oxidationsmittelzubereitung (K2) mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (K2).

In einer besonders bevorzugten Ausführungsform der Erfindung enthält die erfindungsgemäß eingesetzte Oxidationsmittelzubereitung (K2) kein Kationtensid und mindestens ein Öl in einer Gesamtmenge von 0,2 - 50 Gew.-%, besonders bevorzugt 2 - 40 Gew.-%, außerordentlich bevorzugt von 8 - 30 Gew.-%, weiter außerordentlich bevorzugt von 15 - 25 Gew.-%, jeweils bezogen auf das Gewicht der Oxidationsmittelzubereitung (K2).

Das mindestens eine Öl, das in der Oxidationsmittelzubereitung (K2) in einer Gesamtmenge von 0,2 - 50 Gew.-%, bezogen auf das Gewicht der Zubereitung (K2), enthalten ist, ist bevorzugt ausgewählt aus natürlichen und synthetischen Kohlenwasserstoffen, besonders bevorzugt aus Mineralöl, Paraffinölen, $C_{18}$-$C_{30}$-Isoparaffinen, insbesondere Isoeicosan, Polyisobutenen und Polydecenen, $C_8$-$C_{16}$-Isoparaffinen, sowie 1 ,3-Di-(2-ethylhexyl)-cyclohexan; den Benzoesäureestern von linearen oder verzweigten $C_{8-22}$-Alkanolen; Triglyceriden von linearen oder verzweigten, gesättigten oder ungesättigten, gegebenenfalls hydroxylierten $C_{8-30}$-Fettsäuren, insbesondere natürlichen Ölen; den Dicarbonsäureestern von linearen oder verzweigten $C_2$-$C_{10}$-Alkanolen; den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können; den Anlagerungsprodukten von 1 bis 5 Propylenoxid-Einheiten an ein- oder mehrwertige $C_{8-22}$-Alkanole; den $C_8$-$C_{22}$-Fett-alkoholestern einwertiger oder mehrwertiger $C_2$-$C_7$-Hydroxycarbonsäuren; den symmetrischen, unsymmetrischen oder cyclischen Estern der Kohlensäure mit $C_{3-22}$-Alkanolen, $C_{3-22}$-Alkandiolen oder $C_{3-22}$-Alkantriolen; den Estern von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertigen linearen oder verzweigten $C_2$-$C_6$-Alkanolen; Siliconölen sowie Mischungen der vorgenannten Substanzen. In diesem Zusammenhang erfindungsgemäß besonders bevorzugte Öle sind ausgewählt aus Paraffinölen und den Estern der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können, sowie Mischungen hiervon; außerordentlich bevorzugt ausgewählt aus Paraffinöl, Isopropylpalmitat und Isopropylmyristat sowie Mischungen hiervon.

In einer weiteren bevorzugten Ausführungsform der Erfindung enthält die erfindungsgemäß eingesetzte Oxidationsmittelzubereitung (K2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, wobei sich alle Mengenangaben auf das Gewicht der Oxidationsmittelzubereitung (K2) beziehen, und wobei die Zubereitung (K2) keine Kationtenside, keine Öle, kein Polymer mit einem Polymerisierungsgrad von mindestens 200 und kein Polymer mit einem Molekulargewicht von 10000 Dalton oder höher enthält.

Eine weitere erfindungsgemäß bevorzugte Verpackungseinheit (Kit-of-Parts) und ein weiteres erfindungsgemäß bevorzugtes Haarfärbeverfahren sind jeweils dadurch gekennzeichnet, dass die Oxidationsmittelzubereitung (K2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, jeweils bezogen auf das Gewicht der Oxidationsmittelzubereitung (K2), enthält. Eine weitere erfindungsgemäß bevorzugte Verpackungseinheit (Kit-of-Parts) und ein weiteres erfindungsgemäß bevorzugtes Haarfärbeverfahren sind jeweils dadurch gekennzeichnet, dass die Oxidationsmittelzubereitung (K2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, und mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher

enthält.

Es wurde gefunden, dass die Verdickung mit Hilfe der Wechselwirkung zwischen dem Copolymer in dem erfindungsgemäßen Mittel und der vorgenannten Tensid/1-Alkanol-Mischung in der Oxidationsmittelzubereitung (K2) ausreichend ist und durch die Gegenwart eines Polymers mit einem Polymerisierungsgrad von mindestens 200 oder eines Polymers mit einem Molekulargewicht von 10000 Dalton oder höher nicht weiter gesteigert bzw. sogar in ihren Anwendungseigenschaften beeinträchtigt sein kann. Eine weitere erfindungsgemäß bevorzugte Verpackungseinheit (Kit-of-Parts) und ein weiteres erfindungsgemäß bevorzugtes Haarfärbeverfahren sind jeweils dadurch gekennzeichnet, dass die Oxidationsmittelzubereitung (K2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, und mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (K2), enthält.

Eine weitere erfindungsgemäß bevorzugte Verpackungseinheit (Kit-of-Parts) und ein weiteres erfindungsgemäß bevorzugtes Haarfärbeverfahren sind jeweils dadurch gekennzeichnet, dass die Oxidationsmittelzubereitung (K2) mindestens ein Tensid, ausgewählt aus Aniontensiden und Niotensiden sowie Mischungen hiervon, in einer Gesamtmenge von 0,05 - 2 Gew.-%, bevorzugt 0,3 - 1,5 Gew.-%, mindestens ein lineares, gesättigtes 1-Alkanol mit 14 bis 22 Kohlenstoffatomen, ausgewählt aus 1-Tetradecanol (Myristylalkohol), 1-Hexadecanol (Cetylalkohol), 1-Octadecanol (Stearylalkohol) und 1-Eicosanol (Arachylalkohol) sowie Mischungen hiervon, in einer Gesamtmenge von 1 - 5 Gew.-%, bevorzugt 1,5 - 4 Gew.-%, und mindestens ein Öl in einer Gesamtmenge von 0,2 - 50 Gew.-%, bevorzugt 2 - 40 Gew.-%, besonders bevorzugt 8 - 30 Gew.-%, außerordentlich bevorzugt von 15 - 25 Gew.-%, jeweils bezogen auf das Gewicht der Oxidationsmittelzubereitung (K2), aber kein Polymer mit einem Polymerisierungsgrad von mindestens 200 und kein Polymer mit einem Molekulargewicht von 10000 Dalton oder höher enthält.

Als anionische Tenside eignen sich für die erfindungsgemäß verwendeten Oxidationsmittelzubereitungen (K2) alle anionischen Tenside, die vorstehend für die erfindungsgemäßen Mittel insgesamt diskutiert wurden.

Als nichtionische Tenside eignen sich für die erfindungsgemäß verwendeten Oxidationsmittelzubereitungen (K2) alle für die Verwendung am menschlichen Körper geeigneten nichtionischen oberflächenaktiven Stoffe, die vorstehend für die erfindungsgemäßen Mittel insgesamt diskutiert wurden.

Die nachfolgenden Beispiele sollen den Gegenstand der Erfindung darstellen, ohne ihn hierauf zu beschränken.

1.1. Herstellung der Färbemittel

Folgende Farbcremes wurden hergestellt (alle Angaben sind, sofern nichts anderes angegeben ist, in Gewichtsprozent):

| | Creme Nr. 1<br>Komponente (K1) des vierten Gegenstands der Erfindung oder Komponente (K1)-Vergleich | Creme Nr. 2<br>Komponente (K1) des zweiten Gegenstands der Erfindung |
|---|---|---|
| 1-Hydroxyethyl 4,5-diaminopyrazol-Sulfat | 1,50 | 1,50 |
| 4-Amino-2-hydroxytoluol | 0,30 | 0,30 |
| 4-Amino-m-cresol | 0,18 | 0,18 |
| m-Aminophenol | 0,60 | 0,60 |
| Natriumglucoheptonat | - | 0,90 |
| Ammoniumhydroxid | 3,20 | 3,20 |
| Monoethanolamin | 0,60 | 0,60 |
| Kaliumhydroxid | 0,06 | 0,06 |
| Octyldodecanol | 1,60 | 1,60 |
| Cetearylalkohol | 9,60 | 9,60 |

(fortgesetzt)

| | Creme Nr. 1 Komponente (K1) des vierten Gegenstands der Erfindung oder Komponente (K1)-Vergleich | Creme Nr. 2 Komponente (K1) des zweiten Gegenstands der Erfindung |
|---|---|---|
| Glycerylstearat (selbstemulgierend) | 3,00 | 3,00 |
| Ceteareth-20 | 2,40 | 2,40 |
| Natriumlaurethsulfat | 0,90 | 0,90 |
| Natriumcetearylsulfat | 0,50 | 0,50 |
| Ölsäure | 0,30 | 0,30 |
| Natriumsulfit | 0,40 | 0,40 |
| Parfüm | 0,30 | 0,30 |
| Propylenglycol | 0,02 | 0,02 |
| Glycerin | 0,20 | 0,20 |
| Tetranatrium-EDTA | 0,20 | 0,20 |
| Carbomer | 0,12 | 0,12 |
| Ascorbinsäure | 0,05 | 0,05 |
| Natriumsulfat | 0,02 | 0,02 |
| Natriumbenzoat | 0,01 | 0,01 |
| Wasser | 73,94 | 73,04 |

Oxidationsmittel-Komponente (K2)

| | |
|---|---|
| Wasserstoffperoxid | 6,0 |
| Cetearylalkohol | 1,6 |
| PEG-40 Castor Oil | 0,3 |
| Dinatriumpyrophosphat | 0,3 |
| Natriumcetearylsulfat | 0,2 |
| Dinatrium-EDTA | 0,1 |
| Natriumbenzoat | 0,04 |
| Phosphorsäure | 0,03 |
| Wasser | 91,43 |

Komponente (K3) des vierten Gegenstands der Erfindung

| | |
|---|---|
| Natriumglucoheptonat | 8,00 |
| Polyvinylpyrrolidon mit K-Wert 27 - 33 | 10,00 Aktivsubstanz |

(fortgesetzt)

| | |
|---|---|
| Phenoxyethanol | 0,60 |
| Ethylhexylglycerin | 0,09 |
| Keratinhydrolysat | 0,01 |
| Wasser | 81,30 |

Das Vergleichsfärbemittel (V1) wurde durch eine Mischung aus Creme Nr. 1 und der Oxidationsmittel-Komponente (K2) in jeweils gleichen Gewichtsteilen (1:1) hergestellt.

Ein erfindungsgemäßes Färbemittel (E1) gemäß dem ersten Gegenstand der Erfindung wurde durch eine Mischung aus Creme Nr. 2 (als Komponente (K1) des zweiten Gegenstands der Erfindung) und der Oxidationsmittel-Komponente (K2) in jeweils gleichen Gewichtsteilen (1:1) hergestellt.

Ein erfindungsgemäßes Färbemittel (E4) gemäß dem ersten Gegenstand der Erfindung wurde durch eine Mischung aus Creme Nr. 1 (als Komponente (K1) des vierten Gegenstands der Erfindung) mit der Oxidationsmittel-Komponente (K2) und der vorstehend bezeichneten Komponente (K3) im Gewichtsverhältnis (K1) : (K2) : (K3) von 60 : 60 : 1,8 hergestellt.

### 1.2 Ausfärbung auf den Keratinfasern

Normal gebleichte Haarsträhnen der Marke Kerling 7-0, 1 × ultrableached, etwa 1 Gramm) wurden wie folgt behandelt.

Jeweils eines der vorgenannten Färbemittel (V1), (E1) oder (E4) wurde direkt nach seiner Herstellung auf die genannten Haarsträhnen auftragen (Flottenverhältnis 4 Gramm anwendungsbereites Färbemittel (V1), (E1) oder (E4) pro Gramm Haar) und für eine Einwirkzeit von 30 Minuten bei Raumtemperatur (22°C) auf den Haaren belassen. Anschließend wurde das Färbemittel mit Wasser aus den Strähnen ausgespült. Die Strähnen wurden zunächst mit einem Handtuch und dann im kalten Luftstrom getrocknet. Alle Strähnen waren intensiv rot gefärbt.

### 1.3 Messung der Waschechtheit

Für die Evaluierung der Waschechtheit bzw. Waschbeständigkeit der Färbung wurde eine 2 Gew.-%ige wässrige Lösung des nachstehend tabellierten Shampoos in ein Ultraschallbad eingefüllt.

| | |
|---|---|
| Natriumlaurethsulfat | 8,50 |
| Dinatriumcocoamphodiacetat | 0,80 |
| Cocamidopropylbetain | 1,70 |
| Laureth-4 | 0,30 |
| PEG-40 Hydrogenated Castor Oil | 0,10 |
| PEG-7 Glyceryl Cocoate | 0,60 |
| Cocamide MEA | 0,50 |
| Hydrogenated Castor Oil | 0,10 |
| Natriumhydroxid | 0,05 |
| Citronensäure | 0,40 |
| Natriumchlorid | 1,60 |
| Natriumbenzoat | 0,50 |
| Glycoldistearat | 0,60 |
| Jojobaöl | 0,01 |
| Polyquaternium-10 | 0,10 |
| Parfüm | 0,30 |

(fortgesetzt)

| | |
|---|---|
| Wasser | 83,84 |

Die Haarsträhnen wurden bis zu den Enden in diese Lösung eingetaucht und mit Ultraschall behandelt (11 Minuten im Ultraschallbad entsprechen 6 Handwäschen; die Waschlösung wurde nach jedem Waschgang gewechselt). Danach wurden die Strähnen für eine Minute mit handwarmem Leitungswasser gespült und im kalten Luftstrom getrocknet.

Alle Haarsträhnen wurden vor der Färbung, nach der Färbung und nach simulierten 24 Waschgängen, das heißt, nach vier 11-minütigen Ultraschallbädern, an vier verschiedenen Messstellen entlang der Strähne farbmetrisch vermessen.

Zur Bestimmung der Waschbeständigkeit wurden Farbmessungen gemäß dem L*a*b*-Farbsystem durchgeführt.

Für jedes der 3 Färbemittel (V1), (E1) oder (E4) wurden je drei Haarsträhnen verwendet und das arithmetische Mittel jedes Waschzyklus' für alle Haarsträhnen berechnet. Aus den gemessenen L*a*b*-Werten wurde die Farbdifferenz ΔE zwischen der Farbe des gefärbten, unshampoonierten Haars (Wertekombination $L_0^*$, $a_0^*$, $b_0^*$) und der Farbe des gefärbten, shampoonierten Haars nach simulierten 24 Waschgängen (Wertekombination $L_i^*$, $a_i^*$, $b_i^*$) nach folgender Formel berechnet:

$$\Delta E = ((L_i-L_0)^2 + (a_i-a_0)^2 + (b_i-b_0)^2)^{1/2}$$

Je größer der Wert für ΔE ist, desto stärker ausgeprägt ist die Farbdifferenz und desto geringer ist die Waschechtheit oder Waschbeständigkeit der Färbung. Farbdifferenzen mit $\Delta E < 1$ sind für das menschliche Auge nicht wahrnehmbar. Farbdifferenzen mit $\Delta E < 2$ sind lediglich für das geschulte Auge sichtbar. Farbdifferenzen mit $\Delta E > 2$ sind auch für das ungeschulte Auge sichtbar.

Ergebnisse der Farbmessungen zur Waschechtheit:

| Färbemittel | ΔE | ΔΔE zu (V1) |
|---|---|---|
| (V1) | 11.3 | |
| (E1) | 8.8 | 2.5 |
| (E4) | 8.3 | 3.0 |

Die erfindungsgemäßen Färbemittel (E1) und (E4), die jeweils einen Gehalt an Natriumglucoheptonat aufweisen, zeigen gegenüber dem nicht erfindungsgemäßen Färbemittel (V1) ohne Glucoheptonsäure und/oder mindestens eines ihrer physiologisch verträglichen Salze und/oder Lactone nach simulierten 24 Waschgängen, das heißt nach viermal 11 Minuten Ultraschallbad in einer Shampoolösung, eine deutlich geringere Farbdifferenz, also eine deutlich verbesserte Waschechtheit oder Waschbeständigkeit der Färbung.

1.4 Messung der Selektivität

Um festzustellen, ob das anfängliche Farbergebnis entlang der Haarfaser von der Wurzel bis zur Spitze gleichmäßig ist, wurde die wurzelnahe Hälfte der Haarsträhne einmal gebleicht, entsprechend einer mittleren Schädigung, und die andere, spitzennahe Hälfte der Haarsträhne nach zweifacher Glättungsbehandlung zweimal gebleicht, entsprechend einer sehr starken Schädigung.

Zur Beurteilung der Selektivität wurde der Farbabstand ΔE* zwischen der wurzelnahen Strähnenhälfte und der spitzennahen Strähnenhälfte bestimmt.

Je größer der Wert für ΔE ist, desto stärker ausgeprägt ist die Farbdifferenz zwischen beiden Strähnenhälften und desto geringer ist die Homogenität oder Gleichmäßigkeit der Färbung bzw. desto höher ist die Selektivität der Färbung.

Ergebnisse der Farbmessungen zur Selektivität:

| Färbemittel | ΔE | ΔΔE zu (V1) |
|---|---|---|
| (V1) | 2.2 | |
| (E1) | 0.9 | 1.3 |
| (E4) | 1.6 | 0.6 |

Die erfindungsgemäßen Färbemittel (E1) und (E4), die jeweils einen Gehalt an Natriumglucoheptonat aufweisen, zeigen gegenüber dem nicht erfindungsgemäßen Färbemittel (V1) ohne Glucoheptonsäure und/oder mindestens eines ihrer physiologisch verträglichen Salze und/oder Lactone eine geringere Farbdifferenz zwischen Strähnenpartien mit unterschiedlichem Schädigungsgrad, also eine deutlich verbesserte Homogenität oder Gleichmäßigkeit der Färbung und eine verringerte Selektivität der Färbung.

## Patentansprüche

1. Mittel zum oxidativen Färben von keratinischen Fasern, insbesondere menschlichen Haaren, enthaltend in einem kosmetischen Träger

   (A) mindestens ein Oxidationsfarbstoffvorprodukt der Struktur (I) und/oder eines seiner physiologisch verträglichen Salze

   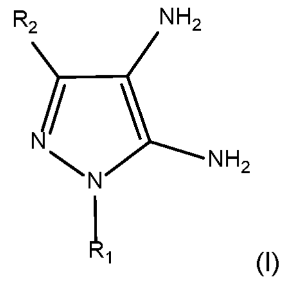

   (I)

   worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, weiterhin
   (B) Glucoheptonsäure und/oder mindestens eines ihrer physiologisch verträglichen Salze und/oder Lactone, und
   (C) mindestens ein Oxidationsmittel, das von Luftsauerstoff verschieden ist,

   **dadurch gekennzeichnet, dass** es mindestens ein Oxidationsfarbstoffvorprodukt der Struktur (I) enthält, in der $R_1$ für eine 2-Hydroxyethyl-Gruppe und $R_2$ für Wasserstoff stehen.

2. Mittel nach Anspruch 1, **dadurch gekennzeichnet, dass** es als physiologisch verträgliches Salz der Glucoheptonsäure Natriumglucoheptonat enthält.

3. Mittel nach einem der Ansprüche 1 bis 2, **dadurch gekennzeichnet, dass** es - bezogen auf sein Gesamtgewicht - Glucoheptonsäure oder mindestens eines ihrer physiologisch verträglichen Salze und/oder Lactone in einer Gesamtmenge von 0,01 bis 2,5 Gew.-%, bevorzugt 0,05 bis 1,5 Gew.-%, besonders bevorzugt 0,1 bis 1,0 Gew.-%, außerordentlich bevorzugt 0,1 bis 0,5 Gew.-% enthält, wobei sich die Mengenangaben auf das Gewicht an freier Glucoheptonsäure in Relation zum Gewicht des erfindungsgemäßen Mittels beziehen.

4. Mittel nach einem der Ansprüche 1 bis 3, **dadurch gekennzeichnet, dass** das mindestens eine Oxidationsmittel, das von Luftsauerstoff verschieden ist, ausgewählt ist aus Wasserstoffperoxid.

**5.** Mehrkomponenten-Verpackungseinheit (Kit-of-parts) zur oxidativen Färbung keratinischer Fasern, umfassend mindestens zwei getrennt voneinander konfektionierte Komponenten (K1) und (K2), wobei

- die erste Komponente (K1) in einem kosmetischen Träger

(A) mindestens ein Oxidationsfarbstoffvorprodukt der Struktur (I) und/oder eines seiner physiologisch verträglichen Salze enthält

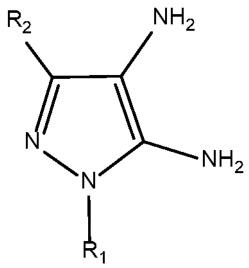

(I),

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, wobei die Komponente (A) gemäß Anspruch 1 ausgewählt ist, weiterhin (C) optional mindestens ein Oxidationsmittel, das ausgewählt ist aus Persalzen, insbesondere Peroxodisulfatsalzen und/oder Peroxomonosulfatsalzen,

- die zweite Komponente (K2)
(C) Wasserstoffperoxid enthält, das in Wasser gelöst ist,

**dadurch gekennzeichnet, dass** mindestens eine der Komponenten (K1) oder (K2)
(B) Glucoheptonsäure und/oder mindestens eines ihrer physiologisch verträglichen Salze und/oder Lactone enthält.

**6.** Mehrkomponenten-Verpackungseinheit (Kit-of-parts) zur oxidativen Färbung keratinischer Fasern, umfassend mindestens drei getrennt voneinander konfektionierte Komponenten (K1), (K2) und (K3), wobei

- die erste Komponente (K1) in einem kosmetischen Träger

(A) mindestens ein Oxidationsfarbstoffvorprodukt der Struktur (I) und/oder eines seiner physiologisch verträglichen Salze enthält

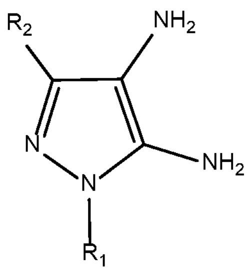

(I),

worin $R_1$ und $R_2$ unabhängig voneinander stehen für Wasserstoff oder eine lineare oder verzweigte C1-C10-Alkylgruppe, die mit ein bis zehn Hydroxyl-Gruppen substituiert sein kann, wobei $R_1$ und $R_2$ nicht gleichzeitig für Wasserstoff stehen, wobei die Komponente (A) gemäß Anspruch 1 ausgewählt ist, weiterhin (C) optional mindestens ein Oxidationsmittel enthält, das ausgewählt ist aus Persalzen, insbesondere Peroxodisulfatsalzen und/oder Peroxomonosulfatsalzen,

- die zweite Komponente (K2)
(C) Wasserstoffperoxid enthält, das in Wasser gelöst ist,
- die dritte Komponente (K3)
(B) Glucoheptonsäure und/oder mindestens eines ihrer physiologisch verträglichen Salze und/oder Lactone

enthält.

7. Verfahren zur oxidativen Haarfärbung von keratinischen Fasern, insbesondere menschlichen Haaren, bei dem ein Mittel gemäß einem der Ansprüche 1 bis 4 auf die Fasern, insbesondere die Haare, aufgetragen wird, dort für eine Zeit von 1 bis 60 Minuten, bevorzugt von 20 bis 45 Minuten, bei Raumtemperatur und/oder bei mindestens 30 °C auf den Fasern verbleibt, die Fasern, insbesondere die Haare, anschließend mit Wasser und/oder einer reinigenden Zusammensetzung gespült werden und gegebenenfalls ein Nachbehandlungsmittel auf die Fasern, insbesondere die Haare, appliziert wird, das gegebenenfalls ausgespült wird, und das Haar anschließend getrocknet wird.

8. Verfahren zur oxidativen Haarfärbung von keratinischen Fasern, insbesondere menschlichen Haaren, bei dem die Komponenten der Kits gemäß Anspruch 5 oder 6 gleichzeitig oder direkt nacheinander ohne Ausspülen auf die Fasern, insbesondere die Haare, aufgetragen werden, dort für eine Zeit von 1 bis 60 Minuten, bevorzugt von 20 bis 45 Minuten, bei Raumtemperatur und/oder bei mindestens 30 °C auf den Fasern verbleiben, die Fasern, insbesondere die Haare, anschließend mit Wasser und/oder einer reinigenden Zusammensetzung gespült werden und gegebenenfalls ein Nachbehandlungsmittel auf die Fasern, insbesondere die Haare, appliziert wird, das gegebenenfalls ausgespült wird, und das Haar anschließend getrocknet wird.

**Claims**

1. An agent for oxidatively dyeing keratinous fibers, in particular human hair, containing, in a cosmetic carrier,

(A) at least one oxidation dye precursor of structure (I) and/or one of its physiologically acceptable salts

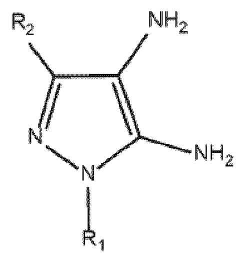

(I)

where $R_1$ and $R_2$, independently of one another, represent hydrogen or a linear or branched C1-C10 alkyl group which may be substituted by one to ten hydroxyl groups, where $R_1$ and $R_2$ do not simultaneously represent hydrogen, and also
(B) glucoheptonic acid and/or at least one of its physiologically acceptable salts and/or lactones, and
(C) at least one oxidizing agent other than atmospheric oxygen,

**characterized in that** it contains at least one oxidation dye precursor of structure (I), in which $R_1$ represents a 2-hydroxyethyl group and $R_2$ represents hydrogen.

2. The agent according to claim 1, **characterized in that** it contains sodium glucoheptonate as a physiologically acceptable salt of glucoheptonic acid.

3. The agent according to one of claims 1 to 2, **characterized in that** it contains - based on its total weight - glucoheptonic acid or at least one of its physiologically acceptable salts and/or lactones in a total amount of 0.01 to 2.5 wt.%, preferably 0.05 to 1.5 wt.%, particularly preferably 0.1 to 1.0 wt.%, extremely preferably 0.1 to 0.5 wt.%, wherein the indication of quantities refers to the weight of free glucoheptonic acid in relation to the weight of the agent according to the invention.

4. The agent according to one of claims 1 to 3, **characterized in that** the at least one oxidizing agent, other than atmospheric oxygen, is selected from hydrogen peroxide.

5. A multicomponent packaging unit (kit-of-parts) for oxidatively dyeing keratinous fibers, comprising at least two components (K1) and (K2) packaged separately from one another, wherein

- the first component (K1) contains, in a cosmetic carrier,

(A) at least one oxidation dye precursor of structure (I) and/or one of its physiologically acceptable salts

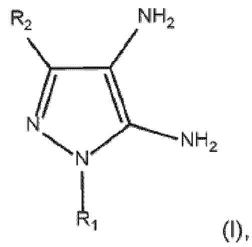

(I),

where $R_1$ and $R_2$, independently of one another, represent hydrogen or a linear or branched C1-C10 alkyl group which may be substituted by one to ten hydroxyl groups, where $R_1$ and $R_2$ do not simultaneously represent hydrogen, wherein component (A) is selected according to claim 1, and also
(C) optionally at least one oxidizing agent selected from persalts, in particular peroxodisulfate salts and/or peroxomonosulfate salts,

- the second component (K2) contains

(C) hydrogen peroxide dissolved in water,
**characterized in that** at least one of the components (K1) or (K2) contains
(B) glucoheptonic acid and/or at least one of its physiologically acceptable salts and/or lactones.

**6.** A multicomponent packaging unit (kit-of-parts) for oxidatively dyeing keratinous fibers, comprising at least three components (K1), (K2) and (K3) packaged separately from one another, wherein

- the first component (K1) contains, in a cosmetic carrier,

(A) at least one oxidation dye precursor of structure (I) and/or one of its physiologically acceptable salts

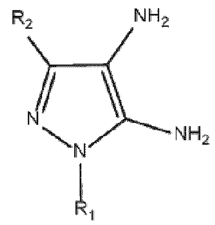

(I),

where $R_1$ and $R_2$, independently of one another, represent hydrogen or a linear or branched C1-C10 alkyl group which may be substituted by one to ten hydroxyl groups, where $R_1$ and $R_2$ do not simultaneously represent hydrogen, wherein component (A) is selected according to claim 1, and also
(C) optionally at least one oxidizing agent selected from persalts, in particular peroxodisulfate salts and/or peroxomonosulfate salts,

- the second component (K2) contains
(C) hydrogen peroxide dissolved in water,
- the third component (K3) contains
(B) glucoheptonic acid and/or at least one of its physiologically acceptable salts and/or lactones.

**7.** A method for oxidatively hair dying keratinous fibers, in particular human hair, in which an agent according to one of claims 1 to 4 is applied to the fibers, in particular the hair, and remains on the fibers for a time of 1 to 60 minutes, preferably 20 to 45 minutes, at room temperature and/or at at least 30 °C, the fibers, in particular the hair, are then rinsed with water and/or a cleaning composition and optionally a post-treatment agent is applied to the fibers, in particular the hair, which is optionally rinsed out, and the hair is then dried.

8. A method for oxidatively hair dying keratinous fibers, in particular human hair, in which the components of the kits according to claim 5 or 6 are applied to the fibers, in particular the hair, simultaneously or directly one after the other without rinsing, and remain on the fibers for a time of 1 to 60 minutes, preferably 20 to 45 minutes, at room temperature and/or at at least 30 °C, the fibers, in particular the hair, are then rinsed with water and/or a cleaning composition and optionally a post-treatment agent is applied to the fibers, in particular the hair, which is optionally rinsed out, and the hair is then dried.

**Revendications**

1. Agent pour la coloration par oxydation de fibres kératiniques, en particulier de cheveux humains, contenant dans un support cosmétique

    (A) au moins un précurseur de colorant d'oxydation de structure (I) et/ou un de ses sels physiologiquement acceptables

    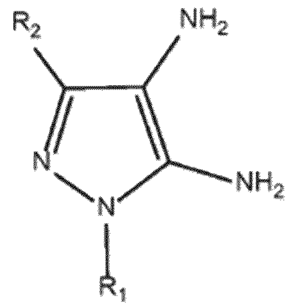

    (I)

    où $R_1$ et $R_2$ représentent indépendamment l'un de l'autre l'hydrogène ou un groupe alkyle en C1-C10 linéaire ou ramifié qui peut être substitué par un à dix groupes hydroxyle, dans lequel $R_1$ et $R_2$ ne représentent pas simultanément l'hydrogène, ainsi que
    (B) de l'acide glucoheptonique et/ou au moins un de ses sels physiologiquement acceptables et/ou une lactone, et
    (C) au moins un agent oxydant différent de l'oxygène atmosphérique.

    **caractérisé en ce qu'**il contient au moins un précurseur de colorant d'oxydation de structure (I), dans laquelle $R_1$ représente un groupe 2-hydroxyéthyle et $R_2$ représente l'hydrogène.

2. Agent selon la revendication 1, **caractérisé en ce qu'**il contient du glucoheptonate de sodium comme sel physiologiquement acceptable de l'acide glucoheptonique.

3. Agent selon l'une des revendications 1 à 2, **caractérisé en ce qu'**il contient, par rapport à son poids total, de l'acide glucoheptonique ou au moins un de ses sels physiologiquement acceptables et/ou une lactone en une quantité totale allant de 0,01 à 2,5 % en poids, de préférence de 0,05 à 1,5 % en poids, de manière particulièrement préférée de 0,1 à 1,0 % en poids, de manière préférée entre toutes de 0,1 à 0,5 % en poids, dans lequel les quantités indiquées se rapportent au poids de l'acide glucoheptonique libre en relation avec le poids de l'agent selon l'invention.

4. Agent selon l'une des revendications 1 à 3, **caractérisé en ce que** l'au moins un agent oxydant différent de l'oxygène atmosphérique est choisi parmi le peroxyde d'hydrogène.

5. Unité d'emballage à plusieurs composants (kit de pièces) pour la coloration par oxydation de fibres kératiniques, comprenant au moins deux composants (K1) et (K2) conditionnés séparément l'un de l'autre, dans laquelle

    - le premier composant (K1) contient, dans un support cosmétique,

        (A) au moins un précurseur de colorant d'oxydation de structure (I) et/ou un de ses sels physiologiquement acceptables

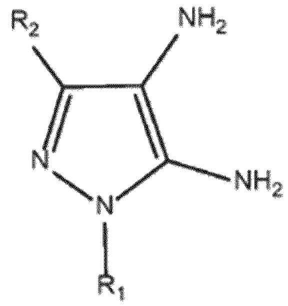

(I)

où $R_1$ et $R_2$ représentent indépendamment l'un de l'autre l'hydrogène ou un groupe alkyle en C1-C10 linéaire ou ramifié qui peut être substitué par un à dix groupes hydroxyle, dans laquelle $R_1$ et $R_2$ ne représentent pas simultanément l'hydrogène, dans laquelle le composant (A) est choisi conformément à la revendication 1, ainsi que
(C) éventuellement, au moins un agent oxydant qui est choisi parmi des persels, en particulier des sels de peroxodisulfate et/ou des sels de peroxomonosulfate,

- le deuxième composant (K2) contient

(C) du peroxyde d'hydrogène qui est dissous dans de l'eau,
**caractérisée en ce qu'**au moins un des composants (K1) ou (K2) contient
(B) de l'acide glucoheptonique et/ou au moins un de ses sels physiologiquement acceptables et/ou une lactone.

**6.** Unité d'emballage à plusieurs composants (kit de pièces) pour la coloration par oxydation de fibres kératiniques, comprenant au moins trois composants (K1), (K2) et (K3) conditionnés séparément les uns des autres, dans laquelle

- le premier composant (K1) contient, dans un support cosmétique,

(A) au moins un précurseur de colorant d'oxydation de structure (I) et/ou un de ses sels physiologiquement acceptables

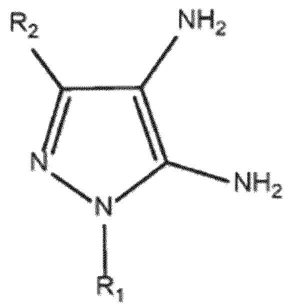

(I),

où $R_1$ et $R_2$ représentent indépendamment l'un de l'autre l'hydrogène ou un groupe alkyle en C1-C10 linéaire ou ramifié qui peut être substitué par un à dix groupes hydroxyle, dans laquelle $R_1$ et $R_2$ ne représentent pas simultanément l'hydrogène, dans laquelle le composant (A) est choisi conformément à la revendication 1, ainsi que
(C) éventuellement, au moins un agent oxydant qui est choisi parmi des persels, en particulier des sels de peroxodisulfate et/ou des sels de peroxomonosulfate,

- le deuxième composant (K2) contient
(C) du peroxyde d'hydrogène qui est dissous dans de l'eau,
- le troisième composant (K3) contient
(B) de l'acide glucoheptonique et/ou au moins un de ses sels physiologiquement acceptables et/ou une lactone.

7. Procédé pour la coloration capillaire par oxydation de fibres kératiniques, en particulier de cheveux humains, dans lequel un agent conformément à l'une des revendications 1 à 4 est appliqué sur les fibres, en particulier les cheveux, reste sur les fibres pendant une durée allant de 1 à 60 minutes, de préférence de 20 à 45 minutes, à température ambiante et/ou à au moins 30 °C, les fibres, en particulier les cheveux, sont ensuite rincées avec de l'eau et/ou avec une composition nettoyante et, éventuellement, un agent de post-traitement est appliqué sur les fibres, en particulier les cheveux, lequel est éventuellement rincé, et les cheveux sont ensuite séchés.

8. Procédé pour la coloration capillaire par oxydation de fibres kératiniques, en particulier de cheveux humains, dans lequel les composants du kit conformément à la revendication 5 ou 6 sont appliqués sur les fibres, en particulier les cheveux, simultanément ou immédiatement les uns après les autres sans rinçage, restent sur les fibres pendant une durée allant de 1 à 60 minutes, de préférence de 20 à 45 minutes, à température ambiante et/ou à au moins 30 °C, les fibres, en particulier les cheveux, sont ensuite rincés avec de l'eau et/ou avec une composition nettoyante et, éventuellement, un agent de post-traitement est appliqué sur les fibres, en particulier les cheveux, lequel est éventuellement rincé, et les cheveux sont ensuite séchés.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1875892 D1
- DE 102006017901 D2
- EP 2471504 A1
- EP 1321131 A2
- WO 2006106366 A1
- WO 2003015734 A1
- JP 2004210700 A
- DE 19756454 A1